(19) 

Europäisches Patentamt
European Patent Office
Office européen des brevets

(11) **EP 4 450 519 A1**

(12) **EUROPEAN PATENT APPLICATION**
published in accordance with Art. 153(4) EPC

(43) Date of publication:
**23.10.2024 Bulletin 2024/43**

(21) Application number: **22797265.0**

(22) Date of filing: **06.10.2022**

(51) International Patent Classification (IPC):
*C07K 16/22* (2006.01)  *A61P 35/00* (2006.01)
*A61K 39/00* (2006.01)

(52) Cooperative Patent Classification (CPC):
**C07K 16/22; A61P 35/00;** A61K 2039/505;
C07K 2317/22; C07K 2317/31; C07K 2317/76;
C07K 2317/92

(86) International application number:
**PCT/CU2022/050011**

(87) International publication number:
**WO 2023/109982 (22.06.2023 Gazette 2023/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.12.2021  CU 20210101**

(71) Applicant: **Centro de Ingeniería Genética y
Biotecnología
La Habana 11600 (CU)**

(72) Inventors:
• **GONZÁLEZ MOYA, Isabel**
  **Playa La Habana 11600 (CU)**
• **BEQUET ROMERO, Mónica**
  **Playa La Habana 10400 (CU)**
• **AYALA ÁVILA, Marta**
  **Playa La Habana 11600 (CU)**

• **GAVILONDO COWLEY, Jorge, Victor
  (deceased) (CU)**
• **MORERA DÍAZ, Yanelys**
  **Playa La Habana 11600 (CU)**
• **MUÑOZ POZO, Yasmiana**
  **Playa La Habana 11300 (CU)**
• **CANAÁN-HADEN AYALA, Camila**
  **Playa La Habana 12100 (CU)**
• **LAMDAN ORDÁS, Humberto**
  **Bauta Artemisa 32400 (CU)**
• **GONZÁLEZ BLANCO, Sonia**
  **Playa La Habana 11600 (CU)**

(74) Representative: **V.O.
P.O. Box 87930
2508 DH Den Haag (NL)**

Remarks:
The complete document including Reference
Table(s) and the Sequence Listing(s) can be
downloaded from the EPO website

(54) **PROANGIOGENIC GROWTH FACTOR-BINDING POLYPEPTIDES**

(57) Polypeptides that bind to proangiogenic growth factors comprising in their amino acid sequence at least one single domain antibody fragment ($V_HH$) and vector encoding a polypeptide that binds to proangiogenic growth factors. A pharmaceutical composition comprising said polypeptide or said vectors encoding a polypeptide that binds to proangiogenic growth factors. Use of the polypeptides that bind to proangiogenic growth factors, or of the vector encoding said polypeptides, for the manufacture of a medicament. Method of treating a pathology that occurs with increased angiogenesis, inflammation, or immunosuppression, in an individual who needs it, by the administration of a therapeutically effective amount of a pharmaceutical composition comprising at least a polypeptide that bind to proangiogenic growth factors or the vector encoding said polypeptide.

**EP 4 450 519 A1**

## Description

## Technical Field

The present invention relates to biotechnology and the field of human health. It provides polypeptides that bind to proangiogenic growth factors such as human vascular endothelial growth factor (VEGF) or basic fibroblast growth factor (bFGF) and inhibit their biological effects. It offers the bases for the generation of pharmaceutical compositions that comprise said polypeptides, which are used in the treatment of pathologies that occur with increased angiogenesis, inflammation, and immunosuppression.

## Prior Art

"De novo" vascularization processes currently occupy a central position in the investigation of multiple diseases, and angiogenesis is now described as an organizing principle in the discovery of new drugs (Folkman, Nat Rev Drug Discov, 2007: 6: 273-86). In the last decade, multiple treatments based on inhibitors of endothelial proliferation and modulators of the assembly and permeability of vascular structures have been approved for use in humans. These treatments could be divided into three fundamental groups: I) those that use small polypeptides, and those of the type of tyrosine kinase inhibitors; II) those based on antibodies, their fragments, and variations; III) those that use the sequence of deoxyribonucleic acid (DNA) coding for the antibodies, its fragments or variations inserted into a viral vector (Apte, et al., Cell, 2019: 176: 1248-64).

Complete monoclonal antibodies and their fragments retaining the antigen-binding site are found in advanced phases of clinical trials. Despite the therapeutic success of monoclonal antibodies, they present a series of drawbacks from the production point of view. Their high molecular weight (150,000 Da), heterotetrameric composition, and the presence of about 15 disulfide bonds prevent their easy production in bacteria and make it difficult in eukaryotic cells. In addition, their high molecular weight impairs tissue penetration, affecting its location at the site of action. (Jovčevska y Muyldermans, BioDrugs, 2020: 34: 11-26). Therefore, technological platforms have been developed that use the antigen-binding site of monoclonal antibodies to generate polypeptides of smaller molecular size.

In this context, single domain antibodies, also known as $V_H H$, are the smallest antibody fragments that exist in nature (15-17 kDa), capable of specifically recognizing an antigen. They are derived from the variable regions of camelid heavy chain antibodies. In addition, these polypeptides are resistant to high temperatures, extreme pH and proteases. (Muyldermans, FEBS J, 2021: 288: 2084-102). The small size of a $V_H H$ can be a disadvantage for therapy. However, the high efficiency and solubility in aqueous environments, the great penetrability into tissues and tumors, and the excellent safety profile and low immunogenicity of $V_H Hs$ make them promising in various immunological applications (Sun, et al., Int J Nanomedicine, 2021: 16: 2337-56).

In the last decade, $V_H Hs$ have demonstrated their potential in different fields of scientific research (Jovčevska y Muyldermans, BioDrugs, 2020: 34: 11-26). Recently, the first $V_H H$ received approval for use in humans and two other polypeptides are in phase II and III clinical trials. (Scully, et al., New England Journal of Medicine, 2019: 380: 335-46). Other molecules progress through the different stages of pre-clinical and clinical trials.

Phage display technology enables efficient stringent selection that retrieves, within a few weeks, multiple high-affinity polypeptides from a large and diverse library (McCafferty, et al., Nature, 1990: 348: 552-4). For $V_H H$ selection $V_H H$ three types of libraries can be used: immune libraries, non-immune (naive), and synthetic libraries. Of these, only the synthetic ones can provide an authentic universal library, which makes it possible to obtain $V_H Hs$ specific for almost any antigen. (Knappik, et al., Journal of Molecular Biology, 2000: 296: 57-86).

Recently, various $V_H Hs$, or polypeptides derived from them have been generated, selected, and evaluated with specificity for mediators of the angiogenic process. A growing number of studies attribute a predominant role to the bFGF/FGFR axis in the mechanism of resistance to anti-VEGF/VEGFR drugs. However, there are no specific $V_H Hs$ for bFGF to date (Wang, et al., Molecular Cancer Therapeutics, 2012: 11: 864-72, Ronca, et al., Expert Opin. Ther. Targets 2015: 19: 1-17, Zahra, et al., Cancers (Basel), 2021: 13: 1422).

Considering the fundamental role of VEGF in the angiogenic process VEGF-specific $V_H Hs$ have been generated, but exclusively from camelid immune libraries. (Farajpour, et al., Journal of Biomolecular Screening, 2014: 19: 547-55, Ebrahimizadeh, et al., Applied Biochemistry and Biotechnology, 2015: 176: 1985-95, Kazemi-Lomedasht, et al., Molecular Immunology, 2015: 65: 58-67). These $V_H H$ have shown their inhibitory effect on the proliferation of endothelial cells and the formation of tubular structures and, in one of them, their antitumor effect was confirmed in the TC-1 model. These $V_H Hs$ recognize VEGF in a denatured conformation, so they must be specific for a linear segment, an element that makes them similar to Bevacizumab. (Kazemi-Lomedasht, et al., Iranian Journal of Basic Medical Sciences, 2017: 399-496).

The fundamental drawbacks for further therapeutic development of these polypeptides, beyond radioactive or fluorescent labeling, are related to:

a) the probable antigenicity of sequences derived from camelids,
b) their low molecular weight that limits bioavailability, due to rapid elimination through the kidneys,
c) the multifactorial nature of the pathologies, which points to the need to target more than one growth factor/receptor system.

Only one of the VEGF-specific V$_H$Hs has been humanized (Kazemi-Lomedasht, et al., Iranian Journal of Basic Medical Sciences, 2018: 21: 260-6). So far the behavior of this protein in *"in vivo"* systems, is unknown. There is at least one anti-VEGF V$_H$H that has been partially humanized and is part of the bispecific construct known as BI836880 (Kovalchuk, et al., Clinical & Experimental Metastasis, 2020: 37: 637-48). This V$_H$H is in phase II clinical studies for age-related macular degeneration (AMD) and metastatic lesions of advanced solid tumors (www.http//clinicaltrials.gov).

To increase the size of V$_H$Hs specific for other molecular targets, bivalent, mono- or bispecific polypeptides have been described that also incorporate binding sites to serum proteins, such as albumin, or the Fc segment of immunoglobulins IgG1, IgG2, and IgG4. For VEGF or bFGF, no strategies have been described that incorporate immunoglobulin Fcs, or binding sites for other proteins, to the V$_H$H-type binding sites.

To date, no humanized monospecific or bispecific multivalent VuH-like polypeptides targeting bFGF have been described, and only two approaches use partially humanized V$_H$Hs for VEGF. Additionally, none of the strategies discussed above has yet taken advantage of the diversity that could be generated by the use of a humanized synthetic library.

Therefore, there is still a need to obtain therapeutic variants with novel and specific binding sites for human VEGF and bFGF, which show increased antiangiogenic, antitumor, antimetastatic, anti-inflammatory, and immune restorative effects. This would allow an additional improvement in their bioavailability and tissue access.

## Details description of the invention

The present invention solves the aforementioned problem by providing polypeptides that bind to proangiogenic growth factors. These polypeptides comprise in their amino acid sequence at least one single domain antibody fragment (V$_H$H) that has an amino acid sequence identified as SEQ ID NO: 23 or SEQ ID NO: 24, or an amino acid sequence that has 95% of identity with these sequences. Said polypeptides are obtained from the sub-libraries generated within the framework of the invention and show physical-chemical properties that make them attractive from the drug formulation and development point of view. In one embodiment of the invention, the proangiogenic growth factor to which said polypeptides bind is human VEGF or bFGF.

In one embodiment of the invention, the human VEGF or bFGF binding polypeptide binds at least two VEGF or bFGF molecules, or one VEGF molecule and one bFGF molecule. In a particular embodiment, the invention provides polypeptides that have an amino acid sequence that is selected from the group consisting of SEQ ID NO: 12, SEQ ID NO: 13, SEQ ID NO: 18, SEQ ID NO: 19, SEQ ID NO: 20, SEQ ID NO: 21, SEQ ID NO: 25, SEQ ID NO: 26, SEQ ID NO: 27 and SEQ ID NO: 28.

The invention shows the obtainment of a polypeptide called Nb-V1 that binds to human and mouse VEGF and inhibits its binding to the VEGF receptor type 2 (VEGFR2), but not to the VEGF receptor type 1 (VEGFR1). The amino acid sequence of said polypeptide is identified as SEQ ID NO: 12. Unlike full-length antibodies, Nb-V1 is preferentially distributed towards tumors, and it was shown to have antiangiogenic properties. Nb-V1 differs from other anti-VEGF antibody fragments in that it does not inhibit VEGF binding to VEGFR1. This receptor has frequently been described as a way of storing VEGF in the extracellular matrix since it does not have effective signal transduction and is also essential in multiple homeostatic processes. (Shibuya, Cell structure and function, 2001: 26: 25-35). Its blockade by other inhibitors of the VEGF/VEGFR system is related to increases in kidney and liver damage, so achieving selective inhibition of VEGFR2 provides an advantage from the toxicological point of view (Sullivan, et al., PLoS ONE 2010: 5: 7-8). VEGFR2 mediates the effects of VEGF on endothelial cell proliferation, migration, and tube formation. (Selvaraj, et al., Cancer Cell, 2015: 27: 780-96), and on the attraction of TReg and MDSC cells, and the induction of senescence of T lymphocytes (Bourhis, et al., Frontiers in Immunology, 2021: 12: 616837)

The invention also shows the obtaining of a V$_H$H called Nb-F3, which binds to human and mouse bFGF, and inhibits its binding to FGFR1, and which is characterized in that its amino acid sequence is identified as SEQ ID NO: 13 This molecule is unique in its class since no V$_H$H with specificity for bFGF has been described. The Nb-F3 polypeptide showed antiangiogenic and antiproliferative properties in vitro.

The monovalent and monospecific polypeptides Nb-V1 and Nb-F3 were obtained in their monomeric form and maintained more than 70% of their antigen recognition and binding blockade to their respective receptors when subjected to 75°C for 2 hours. In addition, the stability of V$_H$H at high concentrations, in a variety of buffers of different pHs, and in simple and complex formulations was also proven. The results obtained for V$_H$Hs Nb-V1 and Nb-F3, in terms of thermal stability from the conformational and functional point of view, indicate the feasibility of obtaining polypeptides with an adequate profile of thermal stability and solubility, from the universal library and the designed maturation libraries. The Nb-V1 and Nb-F3 V$_H$Hs disclosed by the invention are approximately 15 kDa in size. This characteristic offers advantages from tissue penetrability point of view, but conditions its rapid elimination through the kidneys (Cortez-Retamozo, et al., Int J Cancer, 2002: 98: 456-62). Renal elimination imposed the need for repeated administration.

In one embodiment and to increase retention in the circulation, the polypeptide of the invention is a fusion polypeptide comprising: a) an amino acid sequence selected from the group consisting of: SEQ ID NO: 23, SEQ ID NO: 24, SEQ ID NO: 25, SEQ ID NO: 26, SEQ ID NO: 27 and SEQ ID NO: 28 and b) an amino acid sequence corresponding to an albumin binding site or an Fc domain of a human immunoglobulin. In one embodiment of the invention, the Fc domain is from a human IgG1, IgG2, IgG3, or IgG4 immunoglobulin. The design of multimers with spacers (linkers) or hinge regions has been widely described in the literature. These can be synthetic, such as multimers of the amino acid sequence Gly-Gly-Gly-Gly-Ser. They can also be chosen from the group of sequences of hinges and spacers of human origin, or from the camelidae family, which fulfill this function within the immunoglobulins of said species. (Nezlin, The Immunoglobulins, 1998: 3-73, Conrath, et al., Developmental and Comparative Immunology, 2003: 27: 87-103, Saerens, et al., Anal Chem, 2005: 77: 7547-55).

The invention reveals the obtainment of a polypeptide that recognizes two VEGF molecules (bivalent monospecific for VEGF), called Nb-VV6, which inhibits its interaction with VEGFR2 and which has the sequence identified as SEQ ID NO: 20. Also the obtaining of a polypeptide that recognizes two molecules of bFGF (bivalent monospecific for bFGF), called Nb-FF2, which inhibits its interaction with FGFR1, and which has the sequence identified as SEQ ID NO: 21, is revealed. Unlike other molecules in the state of the art Nb-VV6 and Nb-FF2, were obtained in their monomeric form and lost less than 30% of their binding and receptor binding blocking activity when subjected to 2 hours at 75°C. With this strategy, a 3-fold increase in the recognition of the respective ligands was achieved, when compared with the monovalent variants, which correlated with the increase in antiangiogenic effects in vitro. Biodistribution studies demonstrated the accumulation of these monospecific bivalent polypeptides in tumor lesions, similar to that observed for monovalent variants. Analyzes in macular or tumor neovascularization models, in several in vivo studies, indicated a superior effect of these monospecific bivalent polypeptides.

The exclusive sequestration of one of these ligands (bFGF or VEGF) does not definitively neutralize the angiogenic processes associated with the diseases, due to the redundancy of their induction pathways. (Haibe, et al., Frontiers in Oncology, 2020: 10: 221). The inhibition of two or more pro-angiogenic factors is more effective in the prolonged management of macular diseases (Campa, Curr Drug Targets, 2020: 21: 1194-200) and of hepatic tumors (Zahra, et al., Cancers (Basel), 2021: 13: 1422). Considering this, the invention provides bispecific monovalent polypeptides, designated Nb-VF3 and Nb-FV1, which bind both VEGF and bFGF, and inhibit the interaction of these factors with VEGFR2 and FGFR1 receptors, respectively. These bispecific polypeptides are characterized by having an amino acid sequence that is identified as SEQ ID NO: 18 and SEQ ID NO: 19, respectively. Interestingly, it was observed that regardless of the order of the segments corresponding to SEQ ID NO: 23 and SEQ ID NO: 24 in the bispecific polypeptides, they recognize the VEGF and bFGF antigens, and inhibit their interaction with their specific receptors. These bispecific V$_H$Hs conserved the physical-chemical and biological properties described for the monovalent versions of their parental molecules. Analyzes in macular or tumor neovascularization models, in several in vivo studies, indicated the superiority of the bispecific polypeptide Nb-FV1 over the monospecific variant Nb-V1.

Particular reference is made in the invention, to the incorporation into the sequence of the polypeptides identified as SEQ ID NO: 23 to SEQ ID NO: 28 of an Fc (CH1-CH2) fragment of a human IgG1-type immunoglobulin. This element incorporates an effector function, in terms of complement binding, and increases the size of these V$_H$H polypeptides by more than 60 kDa, elements that are desirable for use in neoplastic diseases. (Rath T et al. HHS Public Access. 2016; 35: 235-254). The polypeptides resulting from the addition of the human IgG1 Fc were called Nb-V1_hFc, Nb-F3_hFc, Nb-VV6_hFc, Nb-FF2_hFc, Nb-FV1_hFc and Nb-VF3_hFc, and have the sequences identified as SEQ ID NO: 31 to SEQ ID NO: 36, respectively. The analysis of the recognition of the respective antigens, and of the inhibition of their binding to their receptors, indicated that the addition of this terminal carboxyl segment does not affect the biological properties, as compared to that described for their counterparts without the Fc segment. Additionally, it was shown that the incorporation of this segment significantly increases the half-life of V$_H$H in plasma, which correlates with the antitumor, antiangiogenic, and immunorestorative effects of therapy with them in tumor models. Similarly, the CH2-CH3 segments of other immunoglobulins with less capacity to activate complement can be incorporated, which could be useful in ligand capture, without the parallel induction of inflammatory phenomena.

On the other hand, it has been described that the tracer peptides c-myc and polyhistidine are deleterious to the functionality of small molecules, such as V$_H$Hs. In addition, these tracer segments are preferentially located in the kidney, making this organ a target for toxic effects due to repeated administration. (Huyvetter, et al., Theranostics, 2014: 4). Therefore, the amino acid sequence of polypeptides Nb-V1, Nb-F3, Nb-VV6, Nb-FF2, Nb-FV1, and Nb-VF3 had their carboxyl terminus removed, and the resulting polypeptides were named Nb-V1\cmycH6, Nb-F3\cmycH6, Nb-VV6 \cmycH6, Nb-FF2\cmycH6, Nb-FV1\cmycH6, and Nb-VF3\cmycH6, respectively. These polypeptides possess amino acid sequences that are identified as SEQ ID NO: 23 to SEQ ID NO: 28, respectively. These V$_H$Hs showed similar biological properties to their c-myc and polyhistidine counterparts. The present invention is not restricted to a particular way of obtaining or stabilizing $V_H$Hs. These can be obtained and separated, for example, by protein A affinity chromatography, a widely accepted strategy for the manufacture of recombinant products and which, moreover, ensures the correct folding of the polypeptides that are obtained. Likewise, $V_H$Hs, given their chemical and thermal stability, can be purified using precipitation strategies combined with ion exchange.

In vivo studies in nude animals indicated antitumor and antiangiogenic effects of $V_H$Hs, and their preferential localization toward tumor lesions. The study of vessel density showed a significant reduction in tumors and metastatic foci in animals treated with various variants of $V_H$H. Analysis of antitumor effects in syngeneic mouse models indicated a superior impact of the intervention when comparing immunocompetent versus immunocompromised animals, which could be related to the potential immunorestorative effect of the sequestration of these growth factors. These effects were verified in vitro and in vivo.

For therapeutic applications, the polypeptides of the present invention are administered to an individual in need, in a pharmaceutically effective dose, by routes that are known to one of ordinary skill in the art.

Administration of the polypeptides of the invention significantly reduced tumor growth of neoplasms of various origins, including lung carcinoma, colon carcinoma and renal carcinoma. These results were obtained in models that have been relevant to the translation of the results of other anti-tumor treatments to clinical practice, which indicates the applicability of this strategy to the clinical scenario in cancer treatment. The doses of the polypeptides to be used for each application, depending on the desired effect since an increase in the biological effect has been observed with increasing doses.

The invention also provides a vector that codes for a polypeptide binding to pro-angiogenic growth factors, where the polypeptide has an amino acid sequence selected from the group consisting of SEQ ID NO: 23 to SEQ ID NO: 28 and SEQ ID NO: 31 to SEQ ID NO: 36. This strategy increases the *in vivo* permanence of these polypeptides that bind to proangiogenic growth factors. In this regard, the use of adeno-associated vectors has been described, especially in the suprachoroidal or intravitreal administration of vectors that encode polypeptides with proven benefit in intravitreal administration (Antonio, *et al.,* 2021: 2: 151-7).

The use of the adeno-associated vector AAV2 enabled the *in vitro* and *in vivo* expression of the polypeptides of interest, as shown in Example 14.

The invention discloses a pharmaceutical composition comprising: a) a polypeptide whose amino acid sequence is identified as SEQ ID NO: 23 to SEQ ID NO: 28, SEQ ID NO: 31 to SEQ ID NO: 36 or b) a vector encoding for a polypeptide having an amino acid sequence selected from the group consisting of SEQ ID NO: 23 to SEQ ID NO: 28 and SEQ ID NO: 31 to SEQ ID NO: 36; and a pharmaceutically acceptable excipient.

In the present invention, phosphate buffered saline was preferably used as a vehicle for the polypeptide preparations, but its stability was verified in a wide range of additives. The polypeptides or the vectors can be administered in excipients accepted for pharmaceutical use that are not toxic, and do not have therapeutic effects.

Similarly, the pharmaceutical compositions of the invention are not restricted to a specific administration route, and it is evident that, without specific formulations, $V_H$Hs can be administered intravitreally, intravenously, intraperitoneally, subcutaneously, and intranasally, and generate antiangiogenic, antitumor, anti-inflammatory and immunorestorative effects. Therefore, in an embodiment of the invention, the pharmaceutical composition is formulated for administration by the systemic, mucosal, or intravitreal route.

Another object of the present invention is the use of a polypeptide whose amino acid sequence is identified as SEQ ID NO: 23 to SEQ ID NO: 28, SEQ ID NO: 31 to SEQ ID NO: 36; or a vector encoding a polypeptide having an amino acid sequence selected from the group consisting of SEQ ID NO: 23 to SEQ ID NO: 28 and SEQ ID NO: 31 to SEQ ID NO: 36, for the manufacture of a drug. In one embodiment of the invention, the medicament is useful for the treatment of a pathology that causes increased angiogenesis, inflammation, or immunosuppression. The invention does not restrict the use of the medicament for particular diseases. The invention depicts how drug therapy comprising the polypeptides, or vectors of the invention, is also effective in other contexts, based on the results of modulation of the activation of dendritic cells, T cells, and macrophages obtained in vitro. In a particular embodiment of the invention, the medicament is useful for the treatment of cancer, diabetic retinopathy, macular degeneration, or rheumatoid arthritis.

In another aspect, the invention provides a method for treating a pathology that occurs with increased angiogenesis, inflammation, or immunosuppression in an individual who needs it, characterized by the administration of a therapeutically effective amount of a pharmaceutical composition comprising a polypeptide whose amino acid sequence is identified as SEQ ID NO: 23 to SEQ ID NO: 28, SEQ ID NO: 31 to SEQ ID NO: 36; or the vector that codes for a polypeptide that has an amino acid sequence that is selected from the group consisting of SEQ ID NO: 23 to SEQ ID NO: 28 and SEQ ID NO: 31 to SEQ ID NO: 36. In an embodiment of the invention, in said treatment method, the pathology is cancer, diabetic retinopathy, macular degeneration, or rheumatoid arthritis. In a particular embodiment of the invention, in the treatment method said pharmaceutical composition is administered by the systemic, mucosal, or intravitreal route.

**Detailed embodiments / Examples of Realization**

**Example 1. General Methods**

*Plasmids, microorganisms, enzymes, antibodies, and recombinant proteins*

The pHG-1m, pACR-1Kand pVSJG-huFc vectors (Lamdan, H et al, WO 2008/052489), as well as the strains TG1 (K12_(lac-pro), supE, thi, hsdD5/F' traD36, proA+B+, lacIq, lacZ_M15) and BL21 (F- ompT hsdS (rB-mB-) gal dcm met (DE3)) from *Escherichia coli* were obtained from the CIGB strain collection. The vector pINFUSE was obtained from InvivoGen, EE.UU. KOD Hot Start Master Mix was acquired from Novagen, EE.UU. and the enzymes T4 DNA ligase, alkaline phosphatase, *ApaLI, NotI, NcoI, BgIII, EcoRI, AfIII, Xba*I and Taq polimerase Master Mix were supplied by Promega, EE.UU. Platinum PCR 2X Master Mix was from ThermoScientific, EE.UU.

The filamentous helper phage M13K0, the protein streptavidin and the antibodies anti-Polyhistidine developed in mice, anti-mouse IgG developed in rabbit and anti-human IgG1 developed in sheep, all conjugated to the enzyme horseradish peroxidase (HRP), were supplied by Sigma-Aldrich, USA. HRP-conjugated anti-M13 antibody (directed against phage PVIII protein) was purchased from GE Healthcare, USA. mAb 9E10 (specific for the c-myc peptide) and protein A conjugated to the HRP enzyme was supplied by CIGB, Sancti-Spiritus (Cuba).

Recombinant human bFGF protein, and VEGF receptor type 1 and 2 fused to a human IgG1 Fc (Flt1-Fc and KDR-Fc) were obtained from Sigma, USA. Mouse bFGF and type 1 receptor of bFGF were supplied by SinoBiological, USA. CHO-VEGF protein was obtained in the supernatant of Chinese hamster ovary (CHO) cells, transformed for the secretion of human VEGF 121. This was purified by affinity to metal ions, as described (Sanchez Ramirez, et al., Journal of immunoassay & immunochemistry, 2016: 37: 636-58). GST-hVEGF and GST-mVEGF proteins were produced in E.coli and purified by glutathione affinity, according to the procedures described (Morera, et al., Biotechnology and Applied Biochemistry, 2006: 44: 45-53). The antibody fragment scFv CIGB-166a (Lamdan, et al., Journal of Biotechnology, 2011: 151: 166-74) and the recombinant protein $VEGF_{KDR}$-(Gavilondo, et al., Vaccine, 2014: 32: 2241-50) were provided by the Department of Technological Development of the CIGB, Havana (Cuba). The biotinylated variants of the Bevacizumab antibody and the scFv CIGB166a, named Bevacizumab-biot and CIGB166a-biot, were prepared in the laboratory, following the manufacturer's instructions.

*Selection of phages capable of binding to VEGF or bFGF*

Selection of $V_HH$ -bearing phages from the libraries was directed against two antigens: vascular endothelial growth factor (VEGF) and basic fibroblast growth factor (bFGF), and was carried out as described (Lamdan *et al.,* 2011). Briefly, a concentration of 10 μg/mL was used for GST-hVEGF, and 5 μg/mL for bFGF. To guarantee the selection of clones with higher affinity, the concentration in the coating was reduced by 50% between each cycle. In the case of VEGF, free GST was added (Morera, et al., Biotechnology and Applied Biochemistry, 2006: 44: 45-53) to the phage mixture, at a concentration of 1 mg/mL, in order to increase the specificity during the selection process. The stringency of the selection was ensure by the increase in the number of washes, and the time of the same in each round of selection with PBS-0.1% Tween (PBST) (up to 20 washes per 4 hours). Twenty to 40 clones were randomly selected from rounds 2 and 3 and assessed for antigen immunoreactivity by phage ELISA which is briefly described below. Plasmid DNA from 10-20 positive clones from each library per antigen was isolated and sequenced (Microsith, Alemania).

**Phage ELISA:**

In the case of VEGF, microtiter plates (Costar, USA) were coated with GST-hVEGF (10 μg/mL), while a 5 μg/mL solution was used for bFGF. Coating with the unrelated antigens: Bovine Serum Albumin (BSA) and Glutathione S Transferase (GST) at 10 μg/mL, were used as negative controls. Phage mixtures diluted between 1:10 and 1:1000, were incubated on the plates for 1h at 25°C. Bound phage were detected with a solution containing HRP-conjugated anti-M13 antibody. Peroxidase enzyme activity was detected with substrate solution and absorbance values at 492 nm were determined in a microtiter plate reader (BMG, Clariostar, Germany). Phage mixtures producing an absorbance value for the target antigen, two times higher than the value for the unrelated antigens BSA and GST were considered positive.

*Screening of clones, production and phages characterization*

After infection of E. coli strain TG1 bacteria with selected phages from the second and third cycle of selection, in 96-well cell culture plates, phages carrying $V_HHs$ were produced, according to the described procedure from isolated colonies (Marks, et al., Journal of Molecular Biology, 1991: 222: 581-97). The immunoreactivity of the phage display $V_HHs$ contained in the supernatant of each well was evaluated by ELISA.

The filamentous phages displaying the $V_H$Hs with higher recognition of the antigens were produced and purified by precipitation with polyethylene glycol at a scale of 50 mL, according to the procedure described (Marks, et al., Journal of Molecular Biology, 1991: 222: 581-97). To determine the phage concentration in the resulting preparations, E. coli strain TG1 bacteria in growth exponential phase were infected with the phage preparations, and plated on 2xYTA solid culture medium plates with 2% glucose ( v/v). They were grown for 16 h at 37°C. The concentration of phage in the preparations (colony forming units (cfu)/mL) was determined by colony counting. Phage preparations, at a concentration of $10^{11}$ cfu/mL, were used for evaluation by ELISA.

*Fermentation conditions and purification of $V_H$H fragments and their bivalent and bispecific constructions*

A representative colony of each plasmid construct was grown in LBK liquid medium (8 g/L tryptone, 5 g/L yeast extract, 2.5 g/L NaCl, 50 $\mu$g/mL kanamycin, pH 7.0- 7.5) and, upon reaching an absorbance of 0.8 at 600 nm, 1 mM isopropyl-$\beta$-D-thiogalactopyranoside (IPTG, Sigma) was added to the culture medium for 19 h of induction. The culture was centrifuged at 4500 rpm for 15 min. The periplasmic fraction was obtained from the cell sediment by osmotic shock, after incubation with TES buffer (200 mM Tris-HCl, 1 mM EDTA, 500 mM Sucrose, pH 8) in a 1:20 ratio (g:mL) for 16h at 4°C. Culture supernatant or periplasm extract was diluted in coupling buffer (50mM NaH2PO4, 300mM NaCl, pH 7.8, or 1X phosphate buffer), at a 1:2 ratio, before starting the purification process. Antibody fragments were purified by metal affinity chromatography or protein A affinity chromatography, using the automated AKTAPure system and pre-packed HisTrap FF 5mL or HiTrap rProtein A FF (GE Healthcare, USA) columns, respectively, according to the manufacturer's guidelines. After a change of buffer to PBS 1x, proteins concentration were assessed by Micro Coomassie (BIORAD, USA).

*ELISA for $V_H$Hs recognition of their target antigen*

The 96-well plates were coated with 100 $\mu$L/well of a solution of GST-hVEGF121, GST-mVEGF120, or GST at a concentration of 20 $\mu$g/mL, or with a solution of VEGF$_{KDR}$- at a concentration of 10 $\mu$g/mL, or hFGF-2 or mFGF-2 at a concentration of 5 $\mu$g/mL in 1X PBS for 16 hours at 4°C. After the plates were blocked with PBS-5% skim milk for one hour, 1:2 serial dilutions of the purified $V_H$Hs were added, starting at 2 $\mu$g/mL in PBS-0.5% skim milk. The plates were incubated for 1 hour at 37°C and after several washes, the monoclonal antibody 9E10, specific for c-myc, was added. After one hour of incubation at 25°C the plates were washed thoroughly. The binding of the fragment to the antigen in the solid phase was detected by the peroxidase activity using 100 $\mu$L/well of TMB. The reaction was stopped with 50 $\mu$L/well of stop solution, and absorbance values at 450 nm were determined in a microtiter plate reader.

*Determination of the affinity constant*

Affinity constant (Kaff) for the antibody fragments was determined using the method described by Beatty et al. (Beatty JD, Beatty BG, Vlahos WG. J Immunol Methods. 1987 Jun 26; 100(1-2): 173-9), for which a variation of the previously described antigen recognition ELISA assay was used. In this case, two concentrations were used for coating with each antigen of interest (7.5 $\mu$g/mL and 2.5 $\mu$g/mL for GST-hVEGF; and 5 $\mu$g/mL and 2.5 $\mu$g/mL for bFGF). Kaff was calculated using the formula:

$$K_{aff} = (N\text{-}1) \, / \, 2 \, (N \, [Nb'] \, \text{-} \, [Nb])$$

Where N = [Ag] / [Ag'], [Ag] y [Ag'] correspond to the maximum and minimum concentration of antigen on the coating, and [Nb] and [Nb'] correspond to the concentration of $V_H$H at which 50% recognition is reached for the coating of [Ag] and [Ag'], respectively.

*Competition ELISA between a soluble form of the receptor and the ligand*

The ability of $V_H$Hs to hinder receptors for binding to their ligand was assessed by ELISA, for which 96-well plates (NUNC, USA) were coated with GST-hVEGF or bFGF and blocked as described in the previous section. Serial 1:2 dilutions of the purified $V_H$Hs, from 7500 nM to 46.8 nM, were added and the plates and incubated for 1 h at 37°C. In this step, some wells were left where only PBS-milk was added. Next, Fc-VEGFR2, Fc-VEGFR1 or Fc-FGFR1 were added, and diluted in PBS-milk to a final concentration of 6.25 ng/mL, 50 ng/mL and 100 ng/mL, respectively. After 45 min incubation at 37°C, plates were washed with PBS-0.05% Tween. To detect receptor binding, HRP-conjugated anti-human IgG antibody was added, diluted 1:5000 in PBS-0.5% milk. After incubation for 1 h at 37°C, the plates were washed with PBS-Tween, and TMB was added. The reaction was stopped after 10 min. Wells incubated with the receptors without the addition of the antibody fragments were taken as reference for maximum binding in the absence of competitor. In this assay, in the case of inhibition of the VEGF/VEGFR axis, the CIGB166a antibody fragment was used as a positive control, and an anti-FGF polyclonal antibody was used for the FGF/FGFR axis. The percentage of inhibition of receptor binding to its ligand was calculated as follows:

$$\% \text{ of inhibition} = 100 - (\text{Absorbancia } 450) / (\text{Absorbancia U}m\acute{a}x) * 100$$

***Analysis of tissue and serum concentration of cytokines and growth factors***

Proteins associated with tumor and metastatic tissues were lysed and recovered in M tubes (Miltenyi) and RIPA solution (SIGMA), using an OctoMacs Dissociator, according to the manufacturer's instructions (Miltenyi, Germany). Lysates were centrifuged to remove debris and protein concentration was assessed by BCA (Thermo Fisher Scientific, USA). $V_H$H concentration was evaluated by ELISA, as already described. In order to evaluate the concentration of proangiogenic factors, both in the lysate and in the serum of the animals, the MAGPMAG-24K (Millipore, USA) reagent set was used, and the values were normalized in the case of the tissues to the protein concentration previously determined for the extract. In the case of human tumors implanted in nu/nu nude mice, human VEGF was additionally quantified according to the R&D instructions for reagent set DV001 (R&D, Germany).

**Example 2. Construction of the CDR3 Randomized Humanized Synthetic Library.**

For the humanized $V_H$H synthetic library, the $V_H$H h-NbBCII10 nucleic acid sequence, described by Conrath et al., was selected as a template (Conrath, et al., Antimicrobial agents and chemotherapy, 2001: 45: 2807-12). Thirteen mutations were incorporated in order to humanize the $V_H$H polypeptide without affecting the antigenic recognition capacity, and without altering the physical-chemical properties of the polypeptides in terms of solubility and stability (Vincke, et al., Journal of Biological Chemistry, 2009: 284: 3273-84). The desired sequence was entered into the DNAWorks Online Software (http://helixweb.nih.gov/dnaworks (Hoover, D. y Lubkowski, J., 2002), and a set of 18 oligonucleotides was obtained by optimizing the codon usage for bacteria, in order to synthesize the selected template by means of an overlapping polymerase chain reaction (PCR). To introduce variability in CDR3, a mutagenic oligonucleotide was designed, that includes fragments of the FR3 and FR4 framework regions, keeping the length of 18 amino acids for CDR3 (SEQ ID NO: 1). Codons were formed according to the NNK sequence (where N is an equimolar mixture of A, G, C, and T, and K is an equimolar mixture of G and T). The necessary restriction sites were added to the oligonucleotides in order to perform ApaLI/NotI digestions (SEQ ID NO: 2 and SEQ ID NO: 3), and further incorporate the DNA with the randomized CDR3 into the phagomid vector pHG-1m. For the construction of the synthetic library, which includes diversifications only of CDR3, the protocol described by Kunke was used (Kunkel, Proceedings of the National Academy of Sciences of the United States of America, 1985: 82: 488-92), with some modifications according to Cabanillas-Bernal *et.al.* (Cabanillas-Bernal, et al., PLoS One, 2019: 14: e0213394). The size of the library was determined by counting the total number of colonies obtained after electroporation. For this, serial dilutions between $10^{-2}$ to $10^{-10}$ were made. The presence and size of the inserts in a sample of 12 colonies was determined by PCR with oligonucleotides that hybridize to sequences of the vector pHG-1m that flank the cloned $V_H$H genes. A size of $1.6*10^{12}$ cfu/mL was estimated for the library and a final size of $9.3*10^{11}$ cfu/mL, after adjusting for the 58.3% diversity detected (7 of 12 clones with different sequences of the mold).

**Example 3. Selection of phages displaying specific $V_H$Hs from the CDR3 Universal Library specific for different antigens.**

Two antigens were used for library selection: VEGF and bFGF. Phage selection was performed as described in the Example 1. An enrichment of significance was observed in the recognition of the antigens against which the selection was made, especially by cycles 2 and 3 of the selection procedure. VnH-displaying phages, were produced from isolated colonies, after infection of bacteria of the TG1 strain of E.coli with the selected phages of the third selection cycle, according to the described procedures (Marks, et al., Journal of Molecular Biology, 1991: 222: 581-97). The immunoreactivity of the $V_H$Hs expressed on the phage contained in the supernatant of each well was assessed by a VEGF- and bFGF-specific phage ELISA, as described above. Thus, 20 and 25 positive $V_H$H-producing clones, respectively, with at least 4 times the optical density of the negative control (on GST or BSA), were identified and sequenced to assess diversity (Macrogen, Korea).

*Production and characterization of V$_H$H-carrying phages.*

From the positive clones, the filamentous phages displaying V$_H$H were produced and purified at a scale of 50 mL, according to the described procedure (Marks, et al., Journal of molecular biology, 1991: 222: 581-97). They were used at a concentration of $10^{11}$ cfu/mL for the evaluation by ELISA, of the immunoreactivity profile. Thus, 12 and 18 positive V$_H$H producing clones were identified, respectively, with at least 4 times the OD of the negative control (over GST or BSA). The study was continued with the phage clones that presented greater immunoreactivity to the white antigen. [Clone pHG-1m_Nb-E7 for VEGF and Clone pHG-1m _Nb-A5 for bFGF].

*Obtaining and characterizing the V$_H$H Nb-E7 and Nb-A5.*

The coding sequences for the polypeptides Nb-E7 (SEQ ID NO: 4) and Nb-A5 (SEQ ID NO: 5) were amplified by means of a PCR reaction, starting from the purified DNA of the two selected phage clones (pHG- 1m_Nb-E7 and pHG-1m _Nb-A5) with oligonucleotides SEQ ID NO: 6 and SEQ ID NO: 7.

The PCR products were purified with the QIAquick PCR purification kit (QIAGEN, Germany) and NcoI/NotI digested. The pACR1-K vector was digested with the same enzymes and ligated with the predigested bands using the enzyme T4 DNA ligase. The reaction product was transformed into competent BL21 DE3 cells. A representative colony from each construct was used to express the proteins, as described in the Example 1. The presence of V$_H$H fragments in the periplasmic fraction was evaluated by 15% SDS-PAGE. The result indicated the expression of a protein of apparent molecular weight between 20-15 kDa (when compared to the molecular weight standard), which represents 32% of the total proteins. The identity and integrity of the carboxyl terminus was checked by anti-polyhistidine Western Blot (Sigma, USA).

V$_H$H purification was performed by metal ion affinity as detailed in the Example 1. section, following the manufacturer's recommendations (GE Healthcare, USA). As a result of the process, the V$_H$H called Nb-E7 and Nb-A5 with a purity greater than 95% were obtained. The calculation of affinity for both polypeptides indicated values between $10^{-6}$ and $10^{-7}$ M, which are considered low for this type of polypeptide, so the affinity was matured by randomizing CDR1 and CDR2.

**Example 4. Construction of affinity maturation libraries.**

In the construction of the synthetic libraries for affinity maturation, the plasmid DNA purified from clones pHG-1m_Nb-E7 (SEQ ID NO: 4), which recognizes human VEGF, and pHG-1m _Nb-A5 (SEQ ID NO: 5), which recognizes bFGF, were taken as template. The DNA sequences encoding antibody fragments randomized in the CDR3 were amplified by two-step PCR with the enzyme KOD DNA polymerase (Millipore, USA). Random mutagenesis of CDR1 in reaction A was performed using oligonucleotides SEQ ID NO: 2 and SEQ ID NO: 8, while randomization of CDR2 occurs in reaction B with oligonucleotides SEQ ID NO: 3 and SEQ ID NO: 9. The bands product of these amplifications (A: 150 bp and B: 270 bp) were mixed 1:1 (10 ng of each), and taken as a template for the second reaction in which 400 bp bands were amplified, for each of the parental DNAs, using oligonucleotides SEQ ID NO: 2 and SEQ ID NO: 3. These bands were cloned after ApaLI and NotI digestion in pHG-1m vector, previously digested with the same enzymes. The products were transformed by electroporation into the TG1 strain on 2xYTA solid medium with 2% glucose. Colonies were flushed with 2xYT liquid culture medium, after 24-26 h of growth, to form the maturation sub-library, and stored at -70°C in the presence of 20% glycerol, as a source for the production of filamentous phage displaying antibody fragments.

The size of the sub-libraries was determined as described in Example 2. A size of $7.5*10^{12}$ cfu/mL was estimated for the Nb-E7 mutated sub-library, and a size of $3*10^{12}$ cfu/ mL for the Nb-A5 mutated sub-library.

The selection, production, obtention and characterization of phages expressing V$_H$H with increased recognition of the antigens of interest was performed as described in Example 3. As described for the universal library, an enrichment of phages displaying V$_H$Hs specific for the antigens was observed after the second and third cycle of selection. When analyzing independent clones from the third round of selection, for each antigen, numerous positive clones were detected. The studies were continued with the phage clones that presented greater immunoreactivity against the target antigen [Clone pHG-1m_Nb-V1 for VEGF and Clone pHG-1m_Nb-F3 for bFGF], whose nucleic acid sequences correspond to those sequences identified as SEQ ID NO: 10 and SEQ ID NO: 11, respectively.

**Example 5. Expression in E. coli of the V$_H$H fragments, purification and characterization of the recognition of the specific antigen. Cloning and expression of the V$_H$Hs Nb-V1 and Nb-F3 fragments in the pACR1-K vector.**

The insertion of the V$_H$H sequences in the pACR1-K vector was performed as described in Example 3. A representative colony of each construct was expressed in liquid LBK medium. The presence of VHH fragments in the periplasmic fraction was evaluated by 15% SDS-PAGE. This revealed the expression of proteins of apparent molecular size between 15-20 kDa (when compared to the standard molecular weight), representing 35% of the total proteins. The identity and integrity of the carboxyl terminus was checked by anti-polyhistidine Western Blot (Sigma, USA). The fragments were named Nb-V1 and Nb-F3, and their amino acid sequences were identified as SEQ ID NO: 12 and SEQ ID NO: 13, respectively. The $V_H$Hs were purified by affinity to metal ions, as described in the Example 1, and yields of 7 mg/L and 25 mg/L were obtained when processing the soluble and periplasmic fraction, respectively. These yields exceed those reported by other authors using different humanization strategies, and immune libraries (Publication WO 2003/035694).

***Immunochemical characterization of purified $V_H$H Nb-V1 and Nb-F3 fragments.*** Specific recognition of human and mouse VEGF.

The main characteristic of an antibody and its derived fragments is their recognition of the antigen, which determines their specificity and affinity. Specific binding to VEGF was evaluated as described in the section "ELISA for $V_H$Hs recognition of their target antigen" (Example 1). To compare the capacity of the $V_H$H fragments called Nb-V1 and Nb-E7 for recognizing by the different VEGF-A, the EC50 (nM) representing the concentration value of the $V_H$H that generates 50% recognition, was used. $V_H$H Nb-V1 showed an EC50 for human VEGF of 4.734 nM, three times lower than that detected for Nb-E7 (12.9 nM). This result indicated that the specific recognition of VEGF by the Nb-V1 was improved as compared to the $V_H$H Nb-E7, an element associated with the randomization procedure used for CDR1 and 2, during the affinity maturation process. The results also showed that, unlike other polypeptides in development such as Bevacizumab and CIGB-166a, Nb-V1 recognizes the mouse growth factor with an EC50 of 4,075 nM. Nb-V1 also recognized a mutated variant of VEGF121 in the ELISA system where, in the so-called 80's loop, amino acids R82, K84 and H86 are replaced by E82, E84 and E86. It shares this property with CIGB166a, but differentiates it from Bevacizumab. In addition, $V_H$H Nb-V1 did not recognize other antigens (GST, bFGF) in a similar ELISA format, a fact that points to its antigen specificity.

Competition ELISA between a soluble form of VEGF receptor 1 and 2 (Fc-VEGFR1 and Fc-VEGFR2, respectively) and Nb-V1.

The biological effect of VEGF is a consequence of its binding to two fundamental receptors (VEGFR1 and VEGFR2), so for a polypeptide that binds to VEGF to be effective in the therapeutic setting, it must block these interactions (Shibuya, Cell Structure and Function, 2001: 26: 25-35). The ability of Nb-V1 to compete with receptors 1 and 2 for binding to its ligand (VEGF) was assessed by solid phase ligand competition ELISA, as described above. The results of this assay showed a dose-dependent inhibition of VEGFR2-Fc binding to VEGF by $V_H$H Nb-V1, where 50% inhibition of the signal (IC50) was reached at a concentration of 28.57 nM. An improvement was also detected in this parameter after affinity maturation, since $V_H$H Nb-E7 had an IC50 four times lower in a similar study. Unlike what was reported for Bevacizumab and CIGB166a, the $V_H$H called Nb-V1 did not show dose-dependent inhibition in the case of VEGFR1/VEGF interaction. Only one antibody called Varisacumab, or r84 has been reported with similar characteristics (selectively binding to VEGFR2). Kidney damage is not observed with prolonged use of the r84 antibody, and it has been suggested that the causal element of this lack of renal toxicity is precisely that it does not inhibit VEGFR1 (Sullivan, et al., PLoS ONE 2010: 5: 7-8). Considering this, the $V_H$Hs developed in this invention might induce fewer adverse effects than other similar drugs on the market.

Competition ELISA between $V_H$H Nb-V1 and VEGF-specific antibodies or antibody fragments.

The monoclonal antibody Bevacizumab and the scFv CIGB166a recognize different epitopes within VEGF, and have demonstrated their antiangiogenic effect in various experimental models. Their displacement from their binding to VEGF could be an indication of the potentiality of the new polypeptides to mediate the desired effects in vivo. The ability of anti-VEGF $V_H$H to compete with Bevacizumab antibody and CIGB166a antibody fragment for binding to its ligand was assessed by a variation of the solid phase ligand competition ELISA as described. 96-well plates (NUNC, Germany) were coated with 1 $\mu$g/mL hVEGF121, produced in the CHO cell line. Serial dilutions of purified $V_H$Hs were added, from 15,000 nM to 1,200 nM. Biotinylated Bevacizumab or biotinylated-CIGB166a were then added, diluted in blocking solution to a final concentration of 2.3 ng/mL and 14.3 ng/mL, respectively. To detect the binding of biotinylated antibodies, streptavidin conjugated to HRP was added. The wells incubated with Biotinylated Bevacizumab or biotinylated- CIGB166a, without the addition of the antibody fragments, were taken as reference of maximum binding in the absence of competitor. In this assay, the Fc-VEGFR2 receptor chimera was used as a positive control. The percentage of inhibition of the binding of Bevacizumab, CIGB166a or VEGFR2 to its ligand was calculated as described above in the Example 1. The results of this studies showed that the new $V_H$H called Nb-V1 inhibits, in a dose-dependent manner, the binding of Bevacizumab and CIGB166a to VEGF. A 50% inhibition of the VEGF/Bevacizumab or CIGB166a interaction was observed at 930.9 nM and 67.48 nM, respectively. Although $V_H$H Nb-V1 was shown to recognize a different epitope than Bevacizumab, these results indicate that they may share some amino acid residues withing the VEGF sequence they recognized.

Specific recognition of the basic isoform of human and mouse FGF.

Specific binding to bFGF was evaluated as described in the bFGF recognition ELISAs of the Example 1. For polypeptides Nb-A5 and Nb-F3, it was found that the concentration necessary to produce 50% of the binding to bFGF of human origin is 58.61 nM and 11.26 nM, respectively. As in the case of Nb-V1, a 5-fold increase in recognition was achieved, in correspondence with the affinity maturation process. This $V_H$H also recognized mouse bFGF in the ELISA system, with an EC50 of 11.52 nM, which does not differ significantly from that obtained for human bFGF, due to the high homology between the polypeptides of both species. As in the case of Nb-V1, no recognition of GST or VEGF proteins was observed, thus reinforcing the evidence of the specificity of the binding of the Nb-F3 fragment to its antigen.

Competition ELISA between a soluble form of the hFGF basic isoform receptor 1 (Fc-FGFR1) and $V_H$H Nb-F3.

FGFs mediate their biological action by binding to the extracellular domains of receptor tyrosine kinases called FGFR1-4, present on the surface of endothelial and tumor cells. FGFR1 is the one frequently found on the surface of endothelial cells, and its inhibition in vivo has been shown to significantly reduce tumor burden and vasculature (Ronca, et al., Expert Opin. Ther. Targets 2015: 19: 1-17). The ability of anti-bFGF Nb-F3 $V_H$H to compete with receptor 1 for binding to its ligand was assessed by solid phase ligand competition ELISA. The results of this assay indicated a dose-dependent inhibition of the binding of Fc-FGFR1 to bFGF by $V_H$H Nb-F3, where an IC50 of 134.4 nM was observed. The use of Nb-A5, as a control, resulted in an IC50 5 times higher, an element that corroborates the affinity maturation with the procedure used.

**Example 6. Introduction of modifications to VnHs. Construction of bispecific and bivalent polypeptides with the $V_H$Hs V1 and F3 fragments.**

For the construction of the bispecific and bivalent polypeptides, a two steps PCR was performed, using the enzyme Platinum Hot Start DNA polymerase (Invitrogen, USA) and the corresponding oligonucleotide primers. The oligonucleotides were designed to incorporate in the first PCR, the hinge region of an immunoglobulin called y2C as a spacer between the two $V_H$Hs. In this region, the cysteines were replaced by alanines, in order to avoid the formation of unwanted structures (Hmila, et al., FASEB Journal, 2010: 24: 3479-89). Oligonucleotides SEQ ID NO: 14 and SEQ ID NO: 15 were used to amplify segment 1, and oligonucleotides SEQ ID NO: 16 and SEQ ID NO: 17 were used to amplify segment 2. The products of the first PCR were used as template in the second PCR, to assemble the bispecific and bivalent constructs. The enzyme mentioned above was used, and the primers SEQ ID NO: 14 and SEQ ID NO: 17. The PCR products were purified and digested NcoI/NotI, according to the manufacturer's instructions. The pACR1-K vector was digested with the same enzymes and ligated with the predigested bands using the enzyme T4 DNA ligase (Promega). The nucleotide sequence of the constructs was checked by automated sequencing (Microsynth AG, Germany).

The bispecific Nb-VF3 and Nb-FV1 polypeptides with sequences identified as SEQ ID NO: 18 and SEQ ID NO: 19, and the bivalent Nb-VV6 and Nb-FF2, with sequences identified as SEQ ID NO: 20 and SEQ ID NO: 21, were expressed and purified as described in the Example 1. In all cases, the purity and identity of the proteins were verified by SDS-PAGE 12% and anti-polyhistidine Western Blot. In the four polypeptide preparations, a band with greater than 95% purity, migrating between 30 and 40 kDa, was identified when compared to the weight standard. The experimental mass of the polypeptides and the arrangement of the disulfide bridges were checked by ESI/MS-MS. The experimental and theoretical mass, for each polypeptide coincided, with errors less than 0.0015% in all cases, and the formation of intramolecular bridges between C22-C101 and C168-C247 was verified. Automated Edman sequencing analysis of the amino terminal demonstrated its integrity. The biological activity of the polypeptides was evaluated by ELISA for recognition of the VEGF and bFGF antigens, and by their ability to displace VEGF and bFGF binding to their respective VEGFR2 and FGFR1 receptors. In the case of the bispecific polypeptides designated Nb-FV1 and Nb-VF3, it was shown that the EC50 for each antigen did not vary, when compared to the parental $V_H$Hs Nb-V1 and Nb-F3. Similarly, it was shown that the ability to displace the binding of antigens by their receptors was not affected. On the other hand, for the bivalent polypeptides Nb-W6 and Nb-FF2, the EC50 for VEGF and bFGF decreased 2-fold, when compared to that obtained for Nb-V1 and Nb-F3, respectively (Table 1). In agreement with the increase in recognition, a decrease in the KD of the fragments was observed, calculated according to the methodology described by Beatty et al (Beatty JD, Beatty BG, Vlahos WG. J Immunol Methods. 1987 Jun 26; 100(1-2):173-9). This apparent increase in the affinity of the bivalent polypeptides is attributed to an increase in their avidity when the second domain is added.

**Table 1.** $V_H$H concentrations showing 50% antigen recognition or 50% inhibition of Antigen/Receptor complex formation in ELISA assays.

|  | Nb-V1 | Nb-F3 | Nb-VV6 | Nb-FF2 | Nb-FV1 | Nb-VF3 |
|---|---|---|---|---|---|---|
| EC50 (VEGF) nM | 4.453 | - | 1.895 | - | 4.591 | 4.485 |
| EC50 (bFGF) nM | - | 11.260 | - | 5.862 | 11.465 | 11.321 |
| IC50 (VEGF/VEGFR2) nM | 28.57 | - | 5.6 | - | 28.32 | 28.51 |
| IC50 (bFGF/FGFR1) nM | - | 134.4 | - | 53.52 | 131.32 | 136.26 |

### Removal of c-myc tracer peptide and histidine tag

For the removal of the c-myc tracer peptide and the histidine tail, the bands corresponding to each $V_H$H, or their bispecific or bivalent constructs, were amplified using the enzyme Platinum Hot Start DNA polymerase and the set of primers defined by SEQ ID NO: 15 and SEQ ID NO: 22, which introduces the restriction site of the enzyme BglII towards the 3' end of the polypeptide. The PCR products were purified and digested NcoI/BglII (Promega). The pACR1-K vector was digested with the same enzymes and ligated with the predigested bands using the enzyme T4 DNA ligase. The nucleotide sequence of the $V_H$Hs was verified by automated sequencing (Microsynth AG, Germany). The polypeptides, without the c-myc tracer sequence and without histidine tag, were named Nb-V1\cmycH6 (SEQ ID NO: 23), Nb-F3\cmycH6 (SEQ ID NO: 24), Nb-VV6\cmycH6 (SEQ ID NO: 25), Nb-FF2\cmycH6 (SEQ ID NO: 26), Nb-FV1\cmycH6 (SEQ ID NO: 27) and Nb-VF3\cmycH6 (SEQ ID NO: 28).

Expression of these polypeptides was performed as described. For purification, Protein A affinity chromatography was used, taking advantage of the fact that the $V_H$H antibody fragments retain their ability to bind to this protein (Crauwels M, Van Vaerenbergh N, Kulaya NB et al. New Biotechnology 2020; 57: 20-28). Culture supernatant or periplasm extraction was diluted in PBS 1X, in a 1:2 ratio, before being applied to the pre-packed HiTrap rProtein A FF column (GE Healthcare). The rest of the purification was performed following the manufacturer's recommendations. As a result of this process, polypeptides with a purity greater than 95% were obtained, which migrate between 15-20 kDa (for Nb-V1\cmycH6 and Nb-F3\cmycH6) and 30-40 kDa (for Nb-VV6\cmycH6, Nb-FF2\cmycH6, Nb-FV1\cmycH6 and Nb-VF3\cmycH6) when compared to the molecular weight standard on a 12% SDS-PAGE. Correct removal of tracer sequences was demonstrated by anti-polyhistidine and anti-c-myc Western Blot, and Protein A-HRP was used as a positive control. The analyzes of integrity of the amino terminal did not detect degradations of the same. The ESI/MS-MS results showed a correspondence of the theoretical and experimental size of the polypeptides, with an error ≤0.0019%. In addition, they demonstrated a disulfide bond arrangement similar to that observed for $V_H$Hs Nb-V1, Nb-F3, Nb-W6, Nb-FF2, Nb-FV1, and Nb-VF3. The biological activity of the polypeptides without the c-myc tracer sequence and without the histidine tag was evaluated by ELISAs for the recognition of the VEGF and bFGF antigens, and for the displacement of the binding to their respective VEGFR2 and FGFR1 receptors. Table 2 shows the results, expressed as the concentration values of $V_H$Hs \cmycH6 necessary to achieve 50% antigenic recognition or 50% inhibition of antigen/receptor complex formation. No differences were detected, in terms of binding or inhibition, between $V_H$Hs lacking tracer peptides and their homologous $V_H$Hs.

**Table 2.** Concentration of $V_H$Hs\cmycH6 that represent 50% of the antigenic recognition or 50% of the inhibition of the formation of the Antigen/Receptor complex in ELISA assays.

|  | EC50 (VEGF) | EC50 (bFGF) | IC50 (VEGF/VEGFR2) | IC50 (bFGF/FGFR1) |
|---|---|---|---|---|
| Nb-V1\cmycH6 | 4.64 | - | 28.59 | - |
| Nb-F3\cmycH6 | - | 11.512 | - | 133.86 |
| Nb-VV6\cmycH6 | 1.78 | - | 5.23 | - |
| Nb-FF2\cmycH6 | - | 5.98 | - | 53.66 |
| Nb-FV1\cmycH6 | 4.56 | 11.76 | 28.56 | 131.80 |
| Nb-VF3\cmycH6 | 4.84 | 11.52 | 28.45 | 136.56 |
| **\cmycH6:** no c-myc tracer peptide and no histidine tag | | | | |

***Fusion to the Fc region of a human immunoglobulin.***

The Fc fraction of antibodies mediates effector functions such as antibody-dependent cellular cytotoxicity and complement-dependent cytotoxicity. In order to analyze the potentiality of the polypeptides to promote effector activity, the $V_H$Hs Nb-V1, Nb-F3 and their bispecific and bivalent constructs were inserted into the pVSJG-huFc vector (Publication No. WO 2012/089176). This plasmid was designed to obtain the proteins as fusion polypeptides to the Fc regions of human origin. The oligonucleotide sequences of the $V_H$Hs were amplified, using the enzyme Platinum Hot Start DNA Polymerase and oligonucleotides SEQ ID NO: 29 and SEQ ID NO: 30 for pVSJG-huFc. AflII and XbaI enzyme restriction sites were incorporated. Purified PCR products were digested with these enzymes, as was the pVSJG-huFc vector. The pre-digested bands and vectors were ligated using the enzyme T4 DNA ligase. The nucleotide sequence was checked by automated sequencing (Microsynth AG, Germany), and the new polypeptides were named Nb-V1_hFc (SEQ ID NO: 31), Nb-F3_hFc (SEQ ID NO: 32), Nb-W6_hFc (SEQ ID NO: 33), Nb-FF2_hFc (SEQ ID NO: 34), Nb-FV1_hFc (SEQ ID NO: 35), and Nb-VF3_hFc (SEQ ID NO: 36). Plasmid constructs with the correct sequences were transfected into CHO cells using Superfect reagent (Qiagen, Germany). After selection with the antibiotic G418 (Invitrogen, USA), the clones with the highest expression in the supernatant were selected by antigen recognition ELISAs similar to those already described, where only the detection conjugate for anti-human IgG was changed.

A Protein A affinity chromatography was used for purification. As a result, polypeptides with a purity greater than 95% and with an apparent size between 40-60 kDa were obtained, when compared with the weight standard (Invitrogen, EE. USA), on a 12% SDS-PAGE. Their identity was verified by ELISA. The analyzes of the identity of the amino terminal did not detect its degradations, and the results of ESI/MS-MS determined an agreement between the experimental and theoretical mass for each polypeptide, with errors of less than 0.0012% in all cases. The biological activity of the polypeptides fused to the hFc region was evaluated by ELISAs for the recognition of the VEGF and bFGF antigens, and for the displacement of their binding to their respective VEGFR2 and FGFR1 receptors. Table 3 shows the results of these studies, expressed as concentration values of VnHs_hFc that represent 50% of antigenic recognition, or 50% of the inhibition of the formation of the Antigen/Receptor complex in ELISA-type assays. In this case, a 2-to 4-fold decrease in the EC50s of the fusion polypeptides compared to the homologous $V_H$Hs was observed. This increased recognition corresponds to increased valence since, like a monoclonal antibody, these polypeptides are organized as homodimers. In equal magnitude to the increase in antigen recognition, a decrease in the IC50 of the antigen/receptor interaction was detected.

**Table 3.** Concentration of $V_H$Hs_hFc that represents 50% of the antigenic recognition or 50% of the inhibition of the formation of the Antigen/Receptor complex in ELISA assays.

|  | EC50 (VEGF) | EC50 (bFGF) | IC50 (VEGF/VEGFR2) | IC50 (bFGF/FGFR1) |
|---|---|---|---|---|
| **Nb-V1_hFc** | 1.635 | - | 5.586 | - |
| **Nb-F3_hFc** | - | 4.512 | - | 26.864 |
| **Nb-W6_hFc** | 0.784 | - | 1.123 | - |
| **Nb-FF2_hFc** | - | 2.098 | - | 6.686 |
| **Nb-FV1_hFc** | 1.786 | 4.546 | 5.556 | 26.998 |
| **Nb-VF3_hFc** | 1.884 | 4.652 | 5.456 | 26.756 |

**Example 7. Thermal stability studies of $V_H$Hs.**

$V_H$Hs are known to be resistant to high temperatures, unlike other antibody fragments such as Fabs or scFvs. To determine the resistance of $V_H$Hs Nb-V1, Nb-VV6, Nb-F3, and Nb-FV1 at different temperatures, a study was conducted where 13 $\mu$g of each fragment were incubated at 4, 37, 50, and 75°C. Their immunoreactivity was evaluated by ELISA, after two hours of incubation, and the one obtained for the incubation condition at 4°C was taken as the maximum recognition. In this assay, the CIGB-166a scFv fragment was used as a control. Table 4 shows the recognition capacity of the different fragments by the VEGF or bFGF antigens, in terms of the percentage of the recovered activity, taking as reference the value obtained for the protein stored at -20°C in each case. It was observed that the specific recognition of VEGF by the $V_H$Hs Nb-V1, Nb-VV6, and Nb-FV1 remained close to 70%, even after incubation for two hours at 75°C. This behavior was not observed for the scFv CIGB-166a, which lost antigen recognition. The same resistance to temperature increase, but in terms of recognition of bFGF, was detected for Nb-F3, Nb-FF2 and Nb-FV1.

**Table 4.** Antigenic recognition of V$_H$H Nb-V1 and Nb-F3 after undergoing thermal stress.

| Temperature _time | Recovery of binding activity (% relative to -20°C) | | | | | | |
|---|---|---|---|---|---|---|---|
| | Nb-V1 vs VEGF | Nb-VV6 vs VEGF | Nb-FV1 vs VEGF | Nb-FV1 vs bFGF | Nb-F3 vs bFGF | Nb-FF2 vs bFGF | CIGB-166a vs VEGF |
| 4°C_7D | 100 | 100 | 100 | 96.7 | 100 | 100 | 100 |
| 37°C_2H | 98.0 | 92.6 | 93.6 | 86.3 | 85.5 | 92.5 | 98.0 |
| 50°C_2H | 92.9 | 84.0 | 85.1 | 83.4 | 78.2 | 75.0 | 74.5 |
| 75°C_2H | 83.3 | 75.2 | 72.1 | 75.8 | 76.5 | 73.0 | 6.3 |
| -20°C | 100 | 100 | 100 | 100 | 100 | 100 | 100 |

**Example 8. Ex vivo study of the solubility of the V$_H$H fragments in different buffers and in the vitreous humor of Chinchilla rabbits.**

Taking in consideration the clinical relevance of avoiding aggregates for either intravitreal or systemical administration of antibodies, a preliminary stability study was performed in different buffers. A total of six were tested. Three of this buffers are currently approved for intravitreal administration (Giannos, et al., Pharmaceutical Research, 2018: 1-15):

i. Ranibizumab drug storage formulation (Lucentis B.): 10% α(+)Trehalose, 0.01% Tween 20 and 1.98 mg/mL L-Histidina, pH 5.5.
ii. Formulation A: 10% α(+) Trehalose, 100 mM Phosphate Buffer, 10 mM L-Arginina, 0.3% NaCl and 0.04%, Tween 80, pH 6.78.
iii. Formulation B: 7.5% α(+) Trehalose, 100 mM Phosphate Buffer, 10 mM L-Arginina, 0.3% NaCl and 0,04%, Tween 80, pH 6.78

Other three are buffers for which good V$_H$H stability is expected (Muyldermans, Annual Review of Biochemistry, 2013: 82: 17.1-.23 ):

iv. PBS 1X, pH 7.2
v. Tris 200 mM, pH 8.0
vi. Tris 50 mM, pH 8.0

The V$_H$Hs were concentrated to a maximum of 28 mg/mL, and subsequently adjusted to a concentration of 5 mg/mL. The V$_H$Hs were diluted in the different buffers up to a concentration of 100 and 200 μg/mL and incubated at 4°C for five days. After this period, their immunoreactivity was determined. The CIGB-166a scFv fragment was used as a control. 100% of the activity was recovered, for all the polypeptides under study, in the six buffers, while for the scFv CIGB-166a a 100% recovery was observed only for the Ranibizumab buffer, and losses greater than 20% for the Ranibizumab buffer, rest of the experimental conditions.

The solubility of V$_H$H fragments in the vitreous humor was evaluated in an ex vivo model, considering that the doses to be used in the treatment of macular and cornea conditions range between 0.5 and 2 mg for a final concentration in the vitreous. between 0.125 and 0.5 mg/mL (Giannos, et al., Pharmaceutical Research, 2018: 1-15). The kinetic study at 37°C did not indicate the presence of precipitation phenomena upon inspection under an optical microscope (400X). Analysis of the supernatant, after centrifugation at 6000 rpm, showed no loss of biological activity in terms of ligand recognition (95-100% recovery of activity, for all V$_H$Hs formulations as compared to polypeptides in phosphate buffer showing the maximum recognition). Bevacizumab (Bev) and the CIGB-166a scFv fragment were used as controls in this assay. 100% of the activity was recovered for all polypeptides under study, except for the scFv CIGB-166a, for which a 50% loss of activity was observed, attributable to precipitation.

**Example 9. Evaluation of the effect of the addition of the different polypeptides to in vitro/ex vivo systems.**

The impact of the addition of polypeptides in serial dilutions was analyzed in conditions previously established that duplicates cell proliferation, formation of tubular networks and cells migration, in a VEGF/VEGFR2 or bFGF/FGFR1dependent manner.

*Proliferation studies in endothelial cells:*

Briefly, 6000 HDMEC cells per well were seeded in 100 μL of MCDB131 medium (SIGMA) supplemented with 10% serum, in 96-well plates (COSTAR). After 12 hours, serum was removed from the culture medium by washing and 100 μL of MCDB131 medium were added for 24 hours. The addition of VEGF (5 ng/mL), bFGF (10 ng/mL), or VEGF/bFGF (2.5 ng/mL and 5 ng/mL respectively) induced significant cell proliferation at 72 hours of culture. The $V_H$Hs were added in final concentrations between 300 and 0.5 nM to the proliferation activators in MCDB131 medium, for one hour at 37°C, and added to the cells for a final volume of 200 μL. Cell proliferation was estimated from Alamar Blue metabolism. A 50% reduction in proliferation was observed, for the concentrations shown in Table 5 (IC50).

**Table 5.** Summary of $V_H$H concentrations that inhibit endothelial cell proliferation by 50% (IC50).

| HDMEC Cells | Nb-V1 | Nb-W6 | Nb-F3 | Nb-FF2 | Nb-FV1 | Nb-VF3 | Nb-V1 Nb-F3 | Nb-Neg |
|---|---|---|---|---|---|---|---|---|
| +VEGF | 116 | 30 | >6000 | >6000 | 128.8 | 132 | 121.2 | >6000 |
| +bFGF | >6000 | >6000 | 120,9 | 50 | 122,5 | 120 | 134,5 | >6000 |
| +VEGF +bFGF | 305.9 | 68.1 | 280.5 | 53.1 | 90.4 | 82.8 | 74.2 | >6000 |

The addition of Nb-V1, Nb-VV6, Nb-F3, Nb-FF2, Nb-FV1, and Nb-VF3 resulted in a reduction of VEGF or bFGF dependent proliferation of the microvascular endothelial cell line HDMEC. When using both stimuli, double targeting of VEGF and bFGF systems showed a cooperation effect. These analyzes were replicated with immortalized mouse endothelial cells (H5V) and with primary endothelial cells (HUVEC) and similar results were obtained.

*Studies of the proliferation of immortalized 3T3A31 and NIH3T3 fibroblastoid cells and A549 and B16F10 tumor cells:*

In this case, 3T3A31 and NIH3T3 cells were seeded at a density of 6000 cells per well in 100 μL of DMEM medium, supplemented with 10% FBS, while A549 and B16F10 cells were used at 2000 cells per well. VEGF activation was used as a negative control. The assay was developed differentially, in terms of bFGF concentrations that generate maximal proliferation (100 ng/mL for 3T3A31 and NIH3T3, and 20 ng/mL for A549 and B16F10). A significant reduction in proliferation in response to bFGF was observed for the experimental conditions that included polypeptides with specificity for this growth factor, and the doses for 50% inhibition of proliferation are detailed in Table 6.

**Table 6.** Concentration of $V_H$Hs that inhibit the proliferation of fibroblastoid and epithelial cells by 50% (IC50).

| Cells | Nb-V1 | Nb-F3 | Nb-VV6 | Nb-FF2 | Nb-FV1 | Nb-VF3 | NbN |
|---|---|---|---|---|---|---|---|
| 3T3A31 | >250 | 62.5 | >250 | 28.2 | 55.3 | 49.2 | >250 |
| NIH3T3 | >250 | 91.3 | >250 | 43.1 | 88.5 | 85.6 | >250 |
| HEK-293 | >250 | >250 | >250 | >250 | >250 | >250 | >250 |
| B16F10 | >250 | 95.4 | >250 | 50.2 | 90.3 | 87.1 | >250 |
| A549 | >250 | 85.3 | >250 | 46.2 | 82.9 | 81.3 | >250 |

*Endothelial Network formation studies:*

For these evaluations, HDMEC cells seeded in three-dimensional Matrigel matrix were used (Montesano, R., Pepper, M.S., 1998. En: Little, C.D., Mironov, V., Sage, E.H. (Eds.), Vascular Morphogenesis: In vivo, In vitro, In mente. Birkhauser, Boston, pp. 79-110.). Briefly, 2.5 μL of MATRIGEL per well (SIGMA, USA) were added at 4°C in Terasaki plates, and left to gel at 37°C. 2000 cells per well of CFSE-labeled HDMEC cells were added on 10μl of MCDB131, and the treatments were added in the same volume as shown in Table 7. The results were evaluated at 6 and 24 hours, and were quantified from the images obtained with phase contrast, in an inverted fluorescence microscope (Olympus, Japan). The quantification was performed according to procedures already described, using the ImageJ program (http://rsb.info.nih.gov/ij; NIH, Bethesda, MD, EE.UU.), (D. Guidolin Microvascular Research 67 (2004) 117-124). Table 7 shows an average of the nodes detected per visual field (100X), in response to a fixed concentration of 10 μg/mL of $V_H$H in the presence of 2.5% serum, 100 ng/mL of VEGF or 10 ng /mL of bFGF.

**Table 7.** Average of nodes observed in five fields per experimental replica for each treatment.

| | Nb-V1 300nM | Nb-F3 300n M | Nb-VV6 150nM | Nb-FF4 150nM | Nb-FV3 150nM | Nb-VF3 150nM | NbN 150nM |
|---|---|---|---|---|---|---|---|
| FBS 2.5% | 40 | 29 | 39 | 22 | 28 | 26 | 45 |
| VEGF 100 ng/mL | 9 | 23 | 4 | 19 | 10 | 9 | 41 |
| bFGF 10 ng/mL | 22 | 5 | 8 | 6 | 5 | 5 | 40 |
| MCDB131, FBS 0.1% | 5 | 6 | 4 | 5 | 5 | 4 | 4 |

**Example 10. In vivo studies in Chinchilla rabbits.**

As has already been discussed, one of the potential uses of the $V_H$Hs object of the invention is the use in the treatment of vascular conditions of the macula. In order to analyze the feasibility of using some of them in the treatment of hypervascularization dependent on growth factors VEGF and bFGF, Chinchilla rabbits were used, as described by Morera et al. (Morera, et al., Exp Eye Res, 2014: 122: 102-9). Single injections of 2.5 $\mu$g VEGF or bFGF in 50 $\mu$L were used to induce vascular damage. This procedure has proved to be sufficient to induce an aberrant vascularization pattern in 10 days. 72 hours after the injection, the animals receive a single intravitreal injections of the $V_H$Hs Nb-V1, Nb-F3, Nb-W6 and Nb-FV1, in 50 $\mu$L of saline solution. Surgical microscope examinations, color fundus photography, and fluorescein angiography (FA) were performed one week before and ten days after the first intravitreal injection of the proangiogenic factors. In all cases the rabbits were anesthetized and the pupils dilated as described. At least five photographs were taken in each eye: optic disc, temporal medullary wing, nasal medullary wing, superior retina above the disc, and inferior retina below the disc. An intravenous line was placed in the marginal ear vein, and 0.2 mL of 10% fluorescein was injected. Sequential fundus photographs were taken immediately after fluorescein injection. Late photographs were taken 10 minutes after starting the experiment. In selected cases, photographs of the anterior segment were also taken. Images were processed with Heidelberg Eye Explorer software.

Injection of human VEGF and/or bFGF into the vitreous of the right eye produced retinal neovascularization within 10 days, associated with disc hyperemia, vascular dilatation and tortuosity, and fluorescein leakage in the optic disc, medullary wings, and anterior chamber (AC). The left eye in which only the VHH preparations were injected in the absence of VEGF and/or bFGF and the corresponding images at the start of treatment were used as controls. Semi-quantitative assessments of these effects are shown in Table 8, for each animal and treatment compared to baseline and to the eye receiving no proangiogenic stimulus and the $V_H$Hs. The effects induced by VEGF or bFGF are summarized in a high vascular leak, which disappears in animals treated with a single dose of the specific $V_H$H, which did not present any leak in the optic disc or in the anterior chamber. Retinal vessels became less dilated and tortuous, and retinal capillaries decreased, compared to control group animals for all cases. The analysis of the eyes treated only with the $V_H$H indicated the innocuousness of the treatment, as no inflammatory phenomena associated with their injection were observed.

**Table 8.** Analysis of vascular parameters in the retina of Chinchilla rabbits treated with VEGF or bFGF and the $V_H$Hs

| Animal_ Treatment | Disc hyperemi a | Vascular dilatation and tortuosity | Abnormal vascular pattern | Losses to the optic disc | Neovascula r Membrana |
|---|---|---|---|---|---|
| A1_Nb-Neg | +++ | +++ | +++ | +++ | +++ |
| A2_Nb-Neg | +++ | ++ | ++ | +++ | ++ |
| A2_Nb-Neg | +++ | ++ | ++ | +++ | ++ |
| A1_Nb-V1 | - | + | - | - | - |
| A2_Nb-V1 | + | + | + | - | - |
| A3_Nb-V1 | - | + | - | - | - |
| A2_Nb-W6 | - | + | - | - | - |

In the case of animals A1_Nb-VV6, A3_Nb-W6, A1_Nb-FV1, A2_Nb-FV1, A3_Nb-FV1, no vascular damage of any kind was detected.

**Example 11. Biodistribution.**

The ability of $V_H$Hs to localize to the tumors generated by A673 or A549 cells inoculation was evaluated as compared to a $V_H$H purified from a negative clone for binding to VEGF or bFGF. Human A673 tumor cells express human VEGF and A549 cells express both VEGF and bFGF. The polypeptides were labeled with I131 (Amersham, UK), using the Iodogen method (Fraker PJ, Speck JC Jr. 1978. Biochem Biophys Res Comm 80:849-857), for specific activities of 1.51 MBq/5μg and 1.55 MBq/5μg, respectively. The radiolabeled products were analyzed by thin-layer chromatography to determine protein incorporation, finding values between 92 and 95% of radioactivity in all cases. The ability of radiolabelled products to detect their corresponding antigens (VEGF and human bFGF) was tested in a system where polystyrene immunotubes were coated with recombinant human VEGF isoform 121 (5 μg/mL; Peprotech), or recombinant bFGF (5 μg/mL; SinoBiological, USA). Subsequently, they were blocked, and the sample of the radiolabeled fragments of the corresponding specificity was added to them, adjusted to the quantities that could be trapped by that solid phase. After incubation and washing, it was determined that the solid phase bound between 84.2% and 87.3% of the radioactivity, in all cases, demonstrating that the radiolabeling procedure did not significantly affect the biological activity of the $V_H$Hs.

To study the biodistribution, 126 nu/nu mice were used. The animals were inoculated subcutaneously with $5 \times 10^6$ human tumor cells of the A673 or A549 lines in the right dorsal area. When the tumors reached volumes of $280 \pm 20$ mm$^3$, they were randomized into six groups, of 9 animals for each tumor model, and treatment began. The mice were injected through the tail vein with the radiolabeled product in question, and they were sacrificed in groups of 3 at 24, 48 and 72 hours, surgically removing the tumor and the following normal tissues: spleen, liver, kidney, intestine, muscle, marrow and blood. The accumulation of radioactivity was expressed as a percentage of the injected dose per gram of tissue. Calibration was performed using a standard sample of the injected dose. Radioactivity was determined using a gamma scintillation counter.

Table 9 presents the ratio of radioactivity values in tumor: radioactivity in blood, calculated from the measurements made in these tissues. The study showed that, between 24 and 72 hours, the $V_H$Hs are localized preferentially in the tumor tissue, which is not the case for non-specific Nb-Neg. Greater accumulation of Nb-V1 and Nb-VV6 polypeptides was observed in A673 tumors, and greater concentration of Nb-F3 and Nb-FF2 polypeptides in those caused by the implantation of A549 cells, in correspondence with the high expression of VEGF in A673 and bFGF at A549. The bispecific constructs accumulated in a similar percentage in both types of tumor, and in a greater proportion than the monospecific mono and bivalent variants. No specific deposition of radiolabeled $V_H$H was found in tissues other than the tumor, 48 hours after the VHH was injected.

**Table 9.** Ratio of tumor radioactivity to blood radioactivity, for nude mice transplanted with human A673 or A549 tumor cells..

| $V_H$H | A673 | | | A549 | | |
|---|---|---|---|---|---|---|
| | 24 horas | 48 horas | 72 horas | 24 horas | 48 horas | 72 |
| Nb-V1 | 36.5 | 41.0 | 65.5 | 31.2 | 39.5 | 41.6 |
| Nb-VV6 | 25.5 | 32.0 | 66.5 | 18.8 | 27.3 | 47.2 |
| Nb-F3 | 15.1 | 25.2 | 30.4 | 38.2 | 48.0 | 63.5 |
| Nb-FF2 | 15.1 | 20.2 | 25.4 | 23.2 | 31.9 | 70.4 |
| Nb-FV1 | 45.5 | 56.0 | 85.5 | 40.1 | 55.2 | 83.7 |
| Nb-FV1_Fc | 40.2 | 50.2 | 50.0 | 42.2 | 49.2 | 52.0 |
| Nb-Neg | 0.4 | 1.0 | 0.7 | 0.8 | 1.5 | 0.5 |

The analysis of the concentration of the $V_H$H in the serum of the treated animals showed that 24 hours after the administration of a single dose of 300 μg, equivalent to 15 mg/kg, the concentration of the $V_H$H not fused to Fc (Nb -V1, Nb-F3, Nb-VV6, Nb-FF2 and Nb-FV1) was less than 5 μg/mL. In the case of Nb-FV1_Fc, 132 μg/mL were detected, indicating the superior plasma stability of this construct in maintaining high systemic levels of $V_H$H.

**Example 12. Effect of $V_H$Hs on the maturation of dendritic cells and the activation of T lymphocytes.**

VEGF modulates the maturation and phenotype of dendritic cells and T cells, respectively.(Bourhis, et al., Frontiers in immunology, 2021: 12: 616837). To demonstrate the potential immunomodulatory effect of $V_H$H on cells of these lineages, an in vitro study was performed with (A) CD8+ cells isolated from a negative selection process using magnetic beads (Miltenyi, Germany), from the spleen of an animal 20-week-old C57Bl/6, and (B) 1-week-old mFLT3-differentiated immature dendritic cells isolated from a bone marrow harvest from the same animal.

CD8+ lymphocytes were added to plates coated with anti-CD3 at 10 $\mu$g/mL, and incubated for 48 hours with 50 ng/mL of VEGF (Sigma, USA) preincubated for 2 hours with decreasing concentrations of $V_H$Hs. After this time, the cells were labeled as described by Voron et al. (Voron, et al., J Exp Med, 2015: 212: 139-48), and the results were analyzed in a SYSMEX flow cytometer (Germany). Treatment with increasing concentrations of $V_H$Hs resulted in a significant reduction in all cases of the expression of the senescence marker PD1 in CD8+ cells (p<0.05, Dunnet's a posteriori test), as shown in Table 10A.

**Table 10A.** Analysis of PD1 expression (%) in CD8+ cells treated with VEGF and with the $V_H$Hs.

| Treatment | 10 $\mu$g/mL | 5 $\mu$g/mL | 1 $\mu$g/mL |
|---|---|---|---|
| Nb-V1 | 38±2 | 58±2 | 64±2 |
| Nb-VV6 | 36±5 | 41±4 | 52±5 |
| Nb-FV1 | 40±4 | 55±4 | 63±2 |
| Nb-FV1_Fc | 43±5 | 51±3 | 60±2 |
| Nb-Neg | 65±2 | 67±2 | 68±3 |

Human VEGF and $V_H$Hs mixtures in IMDM medium (Gibco, USA) were preincubated for 2 h for a final concentration of VEGF of 25 ng/mL and 10, 5 and 1 $\mu$g/mL of the $V_H$Hs. Fifteen microliters of these mixtures were added to at total of $10^5$ dendritic cells differentiated with FLT3 per well in 96-well plates. After four hours of incubation, 50 $\mu$L of lipopolysaccharide (SIGMA, USA) were added, for a final concentration of 250 ng/mL. Eighteen hours later, cells were washed and labeled with anti-CD86 PE (Becton Dickinson, USA) and anti-MHCII I-A/I-E-V500 (BD Horizon, USA), and a fixed number of 20,000 cells were analyzed by flow cytometry (SYSMEX, Germany). The addition of increasing concentrations of $V_H$H to the culture of dendritic cells restored the response of these cells to stimulation with lipopolysaccharide, in the presence of VEGF, in a significant and dose-dependent manner in all cases (p<0.05, Dunnet's a posteriori test), as seen in Table 10B.

**Table 10B.** Analyses of mean fluorescence of intensity (MFI) for MHCII and CD86 in dendritic cells treated with VEGF and with $V_H$Hs in the presence of lipopolysaccharide

| | MHCII (MFI) | | | CD86 (MFI) | | |
|---|---|---|---|---|---|---|
| No Stimulus | 4611 | | | 610 | | |
| Stimulus | 17833 | | | 16112 | | |
| +VEGF | 5021 | | | 2000 | | |
| Treatment | 10 $\mu$g/mL | 5 $\mu$g/mL | 1 $\mu$g/mL | 10 $\mu$g/mL | 5 $\mu$g/mL | 1 $\mu$g/mL |
| Nb-V1 | 16950 | 5200 | 4700 | 17200 | 3002 | 1560 |
| Nb-VV6 | 18677 | 11250 | 5112 | 18050 | 6521 | 4200 |
| Nb-FV1 | 17520 | 6000 | 4980 | 17500 | 3200 | 2030 |
| Nb-FV1_Fc | 18800 | 5305 | 5441 | 18000 | 3150 | 1995 |
| Nb-Neg | 4912 | 5141 | 5214 | 1996 | 2050 | 1975 |

## Example 13. Antitumor efficacy of treatment with Nb-V1, Nb-VV6, Nb-F3, Nb-VF3 with or without the Fc fragment in the treatment of solid tumors in mice.

A replica of the study shown in Example 11, in the absence of isotopic labeling of $V_H$Hs, was performed in order to assess the antitumor effect. In all cases groups of 5 mice were used. The animals received the tumor challenge, and according to the characteristics of the model, the treatment began between 3 and 7 days later. The animals were treated for two weeks with 5 mg/kg doses of the $V_H$Hs Nb-V1, Nb-VV6 and Nb-FV1, every 72 hours. At 15 minutes, 2 hours and 72 hours after the first administration, extractions of approximately 50$\mu$L of blood were made for biodistribution analyses. Twenty-two days after the start of treatment, the animals were anesthetized with a lethal dose of ketamine, in order to obtain a total blood sample, and proceed to perfusion of the right atrium with PBS. Serum was separated and primary tumors, liver, kidneys and lungs were weighed separately, depending on the model. The primary tumors, in all cases with diameters less than 10 mm, and the tissues with metastases were divided into three sections that included all the tumor layers. In each case, the tumors or metastases were analyzed by: (a) immunohistochemistry, in order to analyze the vascular density by CD34 selective labeling, (b) flow cytometry, to quantify and characterize the leukocyte infiltrate in the tumor tissue, and (c) ELISA for quantification of $V_H$Hs, cytokines, and growth factors in RIPA tissue lysate and plasma, as described in the Example 1.

**MC-38 Colon Carcinoma Liver Metastases Model:** C57BL/6 animals were challenged by intrahepatic administration of MC-38 mouse metastatic colorectal cancer cells. A laparotomy was performed under anesthesia, and the upper hepatic lobe was exposed, where a total of 10,000 cells were inoculated in 20 μL. Polypeptides were administered intraperitoneally 72 hours after challenge.

**RENCA metastatic renal tumor model:** BALB/c animals were challenged by administration of 20,000 RENCA mouse metastatic renal carcinoma cells. A laparotomy was performed under anesthesia and the kidney was exposed, to inoculate the cells in 20μL, with an ultrafine needle in the subcapsular area. Polypeptides were administered intraperitoneally 72 hours after challenge.

**CT26 Subcutaneous Tumor Model:** BALB/c or nu/nu animals were challenged by subcutaneous administration of 20,000 CT26 mouse colon carcinoma cells. Treatment began peritumorally, when tumor diameters reached between 7 and 8 mm. Treatment were administered every 72 hours, for two weeks.

**Subcutaneous A673 or A549 Tumor Model:** Nu/nu animals were challenged by subcutaneous administration of 5×10⁶ human A673 or A549 cells. Treatment began peritumorally, when tumor diameters between 7 and 8 mm were reached. It was administered every 72 hours for two weeks,. In these models, in particular, the $V_H$H variant Nb-FV1-hFc was evaluated at the same doses and route.

**CT26 Lung Implant Model:** BALB/c animals were challenged by retro-orbital administration of 20,000 CT26 cells, in 20 μL, with an ultrafine needle. Treatment was started by the nasal route 72 hours after the challenge, with 25 μL per nostril, for a total dose of 15 mg/kg.

## Histopathological analyses

To investigate whether $V_H$H treatment affected tumor angiogenesis and tumor cell survival, tumor sections were analyzed by hematoxylin/eosin staining, and by immunofluorescence with a mouse anti-CD34 antibody, as described by Adinolfi and collaborators (Adinolfi, *et al.,* 2012: 2957-69). The results were expressed as the mean vascular density (MVD) as a function of the number of vessels per mm².

Tables 11, 12, and 13 show a summary of the results of tumor growth inhibition, histopathological studies of tumor vascularization and concentrations, in particular of VEGF in tumor lysates, relative to the negative control.

**Table 11.** Percentage of growth inhibition of neoplastic lesions compared to the control group treated with Nb-Neg.

| | C57B1/ 6 | BALB/c | | | | nu/nu | | |
|---|---|---|---|---|---|---|---|---|
| | MC38 Liver | RENCA Splee n | RENC A Lung | CT26 lung | CT26 SC | CT26 SC | A673 SC | A549 SC |
| **Nb-V1** | 44±2.3 | 32±1.2 | 28±1.7 | 50±2.8 | 48±3.3 | 31±3.5 | 30±5.6 | 35±6.4 |
| **Nb-VV6** | 55±3.8 | 41±3.1 | 54±2.7 | 60±3.9 | 65±3.9 | 38±5.3 | 45±7.8 | 40±8.5 |
| **Nb-FV1** | 72±5.3 | 60±4.4 | 65±5.6 | 75±6.3 | 77±5.1 | 45±3.8 | 58±2.3 | 36±3.6 |
| **Nb-FV1_hFc** | | | | | | | 23±4.2 | 15±4.6 |

**Table 12.** Microvessel density (MVD, CD34+ vessels/mm2) in neoplastic lesions from treated and untreated animals.

| | C57B1/6 | BALB/c | | | | nu/nu | | |
|---|---|---|---|---|---|---|---|---|
| | MC38 Liver | RENCA Spleen | RENCA Lung | CT26 lung | CT26 SC | CT26 SC | A673 SC | A549 SC |
| | **Median (range, min-max) n=5, 10 fields per tumor/metastasis** | | | | | | | |
| **Nb-Neg** | 60 ± 22 | 71 ± 23 | 80 ± 25 | 75 ± 20 | 65± 24 | 63± 22 | 91 ± 21 | 82 ± 23 |
| **Nb-V1** | 35 ± 13 | 34 ± 14 | 35 ± 13 | 40 ± 15 | 30 ± 12 | 33 ± 16 | 45 ± 14 | 40 ± 17 |
| **Nb-VV6** | 25 ± 7 | 36 ± 5 | 25 ± 6 | 32 ± 7 | 20 ± 5 | 24 ± 10 | 33 ± 6 | 29 ± 8 |
| **Nb-FV1** | 10 ± 4 | 15 ± 3 | 20 ± 4 | 19± 2 | 10 ± 2 | 15 ± 3 | 25 ± 4 | 21 ± 2 |

(continued)

| | C57B1/6 | BALB/c | | | | nu/nu | | |
|---|---|---|---|---|---|---|---|---|
| | MC38 Liver | RENCA Spleen | RENCA Lung | CT26 lung | CT26 SC | CT26 SC | A673 SC | A549 SC |
| Nb-FV1_hF c | | | | | | | 15 ± 4 | 11 ± 2 |

**Table 13.** VEGF expression in neoplastic tissue from treated and untreated animals.

| Treatment | C57B1/6 | BALB/C | | | | nu/nu | | |
|---|---|---|---|---|---|---|---|---|
| | MC38 Liver | RENCA Spleen | RENCA Lung | CT26 lung | CT26 SC | CT26 SC | A673 SC | A549 SC |
| | Mouse VEGF (pg/mg protein) | | | | | | Human VEGF | |
| Nb-Neg | 440±50 | 640±51 | 560±55 | 580±62 | 988±102 | 956±93 | 999±123 | 745±101 |
| Nb-V1 | 190±22 | 240±31 | 185±32 | 198±31 | 455±33 | 440±32 | 555±54 | 325±73 |
| Nb-VV6 | 101±12 | 125±23 | 99±18 | 75±16 | 165±25 | 145±23 | 265±32 | 185±25 |
| Nb-FV1 | 54±8 | 62,2±7 | 25±9 | 32±4 | 48±3 | 31±7 | 88±13 | 75±13 |
| Nb-FV1_hFc | | | | | | | 24±7 | 15±6 |

A significant antitumoral effect was observed after the use of Nb-V1 by the different routes of administration (intraperitoneal, peritumoral and nasal), in the tumor models studied ($p < 0.05$ Student's t-test) (Table 11). This effect correlated with reduced vascularity and tissue levels of mouse VEGF in immunocompetent animals, and human in nude animals ($p < 0.05$; Pearson's test for all cases) (Table 12 and Table 13). The use of similar doses of Nb-VV6 resulted in a significantly superior effect, compared to Nb-V1, consistent with the higher affinity and avidity of this $V_HH$ for VEGF and its longer systemic half-life ($p < 0.05$; Student's t-test in all cases). The use of Nb-FV1, on the other hand, resulted in an increase in the antitumor and antiangiogenic effect with respect to Nb-V1 and Nb-VV6, due to the incorporation of a second molecular target and the known cooperativity of the inhibition of VEGF/VEGFR and bFGF/FGFR systems in the induction of the angiogenic event (Asahara, et al., Circulation, 1995: 92: II365-71). The incorporation of the CH2-CH3 segment of human IgG1 into Nb-FV1 resulted in an increase in the antitumor and antiangiogenic effect, with respect to the use of Nb-FV1 in the subcutaneous tumor models in which it was tested ($p < 0.05$, Student's t-test).

The comparison of the treatment in the CT26 subcutaneous model in immunocompetent animals, with respect to immunocompromised, indicated a superior antitumor effect in the former. This fact could be related to the immunopotentiating and anti-inflammatory effects described for anti-VEGF and anti-bFGF treatments. Previously, it was shown that the addition of the $V_HH$s Nb-V1, Nb-F3 and Nb-FV1, to in vitro systems for phenotypic analysis of T cells and dendritic cells, inhibits the effects of VEGF and bFGF on the functionality of these leukocytes (Example 12).

Analysis of CT26 tumor-infiltrating CD3+ cells in BALB/c mice indicated that, VHH treatment significantly succeeded in immunorestoring T cells (Table 14A). In this same sample, the population of F4/80+GR1- macrophages was also analyzed in terms of the expression of the M1 phenotype marker: MHC II (expressed as MFI). The results indicate that all treatments increased the functionality of M1-type macrophages, with respect to the group that received treatment with Nb-Neg in each experimental model ($p < 0.05$ Dunnet's a posteriori test) (Table 14B). A higher increase was detected in the groups in which Nb-FV1 was used as treatment, an element that could be associated with the fact that bFGF inhibition additionally contributes to the rescue of immunosuppression as reported by Im et al.(Im, et al., Nature communications, 2020: 11: 1-14).

**Table 14A.** Analysis of the senescence of the infiltrating T cell population in tumors according to the percentage of expression of the PD1 marker.

| Treatment | C57B1/6 | BALB/C | | | |
|---|---|---|---|---|---|
| | MC38 Liver | RENCA Spleen | RENCA Lung | CT26 Lung | CT26 SC |
| | PD1 expression (%) in CD3+ tumor population | | | | |
| Nb-Neg | 30±5 | 40±5 | 42±5 | 58±6 | *48±10* |
| Nb-V1 | 10±2 | 15±3 | 12±32 | 19±31 | *15±33* |

(continued)

| Treatment | C57B1/6 | BALB/C | | | |
|---|---|---|---|---|---|
| | MC38 Liver | RENCA Spleen | RENCA Lung | CT26 Lung | CT26 SC |
| | PD1 expression (%) in CD3+ tumor population | | | | |
| Nb-VV6 | 7±12 | 9±23 | 7±18 | 7±16 | 5±2 |
| Nb-FV1 | 5±8 | 6±7 | 4±9, | 3,2±4 | 2±3 |

**Table 14B.** Analysis of the expression of MHC II in the fraction of macrophages infiltrating tumor lesions.

| Treatment | C57B1/6 | BALB/C | | | |
|---|---|---|---|---|---|
| | MC38 Liver | RENCA Spleen | RENCA Lung | CT26 Lung | CT26 SC |
| | MFI* for MHC II in F4/80+ Gr1- cells | | | | |
| Nb-Neg | 6000 | 5200 | 5305 | 5000 | 5523 |
| Nb-V1 | 10477 | 11250 | 15677 | 16250 | 18677 |
| Nb-VV6 | 11567 | 13526 | 16699 | 17855 | 19523 |
| Nb-FV1 | 16800 | 18542 | 19565 | 20541 | 23000 |
| * The average MFI of 5 samples evaluated per model is shown. | | | | | |

The analysis of the concentration of the VHHs in the serum of the BALB/c and nude animals, treated by the peritumoral route, showed that the half-life of the $V_H$Hs Nb-V1, Nb-W6 and Nb-FV1 was lower than the 2 hours, with a significant increase in the remaining concentration in plasma for bivalent or bispecific $V_H$H compared to Nb-V1. The analysis of the hFc fusion variant of Nb-FV1, on the other hand, showed a stability in plasma greater than 3 days, so its concentrations increased due to the administration of multiple doses (Table 15).

**Table 15.** Evaluation of the plasma concentration of $V_H$H ($\mu$g/mL).

| Treatment | BALB/C | | | nu/nu | | | |
|---|---|---|---|---|---|---|---|
| | 15 min | 2 h | 3 días | 15 min | 2 h | 3 días | 22 días |
| Nb-Neg | 90 | 25 | <5 | 90 | 25 | <5 | <5 |
| Nb-V1 | 92 | 27 | <5 | 92 | 27 | <5 | <5 |
| Nb-VV6 | 98 | 42 | <5 | 98 | 42 | <5 | <5 |
| Nb-FV1 | 89 | 41 | <5 | 89 | 41 | <5 | <5 |
| Nb-FV1_hFc | - | - | - | 90 | 89 | 88 | 300 |

**Example 14. In vitro functional evaluation of gene transfer of $V_H$Hs with viral vector.**

One of the delivery strategies included in clinical trials in current therapeutics for AMD and cancer is temporary gene transfer of therapeutic polypeptides. To this end, the sequence corresponding to Nb-W6_hFc and Nb-FV1_hFc was amplified with a PCR, using oligonucleotides SEQ ID NO: 37 and SEQ ID NO: 38, according to the protocols already described. After digesting NotI/PmeI, both the pAAVK-E!F2-MCS vector and the band were ligated and transformed into the DH5α strain. After verification of the sequence of the new vectors: pAAVK-EIF2-Nb-VV6_hFc and pAAVK-EIF2-Nb-FV1_hFc, these were used to package the viral particles in the AW2 system in HEK-293 cells, according to the manufacturer's instructions (GENEMEDI, China). Viral particles were isolated and concentrated from HEK293 cells co-transfected with 72 hours of culture, as described by Hughes et al. (Hughes, et al., Molecular Therapy: Methods & Clinical Development, 2019: 13: 86-98). After titration by qPCR, they were stored at -80°C until use (Aurnhammer, et al., Hum Gene Ther Methods, 2012: 23: 18-28).

The infectivity and the ability to successfully produce the polypeptides were evaluated in in vitro and in vivo assays. HEK-293 cells were seeded at a density of $5\times10^5$ cells per well in the 6-well plate format. After 24 h, $8\times10^8$ UG (Genome Units) in 1 mL of serum-free medium supplemented with 200 nM glutamine were added to the culture. They were left in contact for one hour, before adding 1 mL of medium supplemented with 20% FBS and glutamine. Culture supernatants were collected after 72 h of incubation at 37°C and 5% CO2. For evaluation of in vivo translation, C57BL/6 mice were given a single injection of $8 \times 10^8$ UG by the intramuscular route. Fourteen days after challenge, the total amount of $V_HH\_hFc$ in tissue was measured by ELISA, as described in General Methods, using a standard curve of each $V_HH\_hFc$ purified and characterized from CHO cells.

Expression of Nb-W6_hFc and Nb-FV1_hFc polypeptides was detected in the culture supernatant of infected HEK-293 cells (125 μg/mL and 220 μg/mL, respectively). $V_HH$ concentrations between 50 and 100 μg per processed gram were detected in muscle tissue. The concentrations detected in both expression formats coincide with those reported for each case in the literature for viral particles packed in the AW2 system and in all cases showed their functionality in terms of binding to specific antigens and blocking their binding to their receptors.

**Claims**

1. A polypeptide that binds to proangiogenic growth factors that comprises in its amino acid sequence at least one single domain antibody fragment (VHH) that has an amino acid sequence identified as SEQ ID NO: 23 or SEQ ID NO: 24, or an amino acid sequence that has 95% identity to the amino acid sequences identified as SEQ ID NO: 23 and SEQ ID NO: 24.

2. The polypeptide of claim 1 wherein the proangiogenic growth factor is human vascular endothelial growth factor (VEGF) or basic fibroblast growth factor (bFGF).

3. The polypeptide of claim 2 that binds to more than one human VEGF or bFGF molecule.

4. The polypeptide of claim 3 that binds at least: i) two VEGF molecules, ii) two bFGF molecules, or iii) one VEGF molecule and one bFGF molecule.

5. The polypeptide of claim 1 having an amino acid sequence selected from the group consisting of SEQ ID NO: 12, SEQ ID NO: 13, SEQ ID NO: 18, SEQ ID NO: 19, SEQ ID NO: 20, SEQ ID NO: 21, SEQ ID NO: 25, SEQ ID NO: 26, SEQ ID NO: 27 and SEQ ID NO: 28.

6. The polypeptide of claim 1 which is a fusion polypeptide that comprises an amino acid sequence selected from the group consisting of: SEQ ID NO: 23, SEQ ID NO: 24, SEQ ID NO: 25, SEQ ID NO: 26, SEQ ID NO: 27 and SEQ ID NO: 28 and an amino acid sequence corresponding to an albumin binding site or a Fc domain of a human immunoglobulin.

7. The polypeptide of claim 6 wherein the Fc domain is from a human immunoglobulin IgG1, IgG2, IgG3 or IgG4.

8. The polypeptide of claim 7 that has an amino acid sequence that is identified as SEQ ID NO: 31 to SEQ ID NO: 36.

9. A vector encoding a polypeptide that binds to proangiogenic growth factors wherein the polypeptide has an amino acid sequence that is selected from the group consisting of SEQ ID NO: 23 to SEQ ID NO: 28 and SEQ ID NO: 31 to SEQ ID NO: 36.

10. A pharmaceutical composition comprising the polypeptide of any one of claims 1 to 8 or the vector of claim 9 and a pharmaceutically acceptable excipient.

11. The composition of claim 10 which is formulated for administration by the systemic, mucosal, or intravitreal route.

12. Use of the polypeptide of any of claims 1 to 8 or of the vector of claim 9 for the manufacturing of a medicament.

13. The use of the polypeptide according to claim 12, wherein the drug is useful for the treatment of a pathology that causes increased angiogenesis, inflammation or immunosuppression.

14. The use of the polypeptide according to claim 13, where the drug is useful for the treatment of cancer, diabetic retinopathy, macular degeneration or rheumatoid arthritis.

15. A method of treating a pathology that occurs with increased angiogenesis, inflammation or immunosuppression in an individual who needs it, **characterized in that** a therapeutically effective amount of a pharmaceutical composition comprising the polypeptide of any of claims 1 to 8 or the vector of claim 9.

16. The treatment method according to claim 15, wherein the pathology is cancer, diabetic retinopathy, macular degeneration, or rheumatoid arthritis.

17. The treatment method according to claim 15, wherein said pharmaceutical composition is administered by systemic, mucosal or intravitreal route.

# INFORME DE BÚSQUEDA INTERNACIONAL

| | |
|---|---|
| | Solicitud internacional N° |
| | **PCT/CU2022/050011** |

## A. CLASIFICACIÓN DEL OBJETO DE LA SOLICITUD

**INV. C07K16/22    A61P35/00    A61K39/00**
**ADD.**

De acuerdo con la Clasificación Internacional de Patentes (CIP) o según la clasificación nacional y CIP.

## B. SECTORES COMPRENDIDOS POR LA BÚSQUEDA

Documentación mínima buscada (sistema de clasificación seguido de los símbolos de clasificación)

**C07K  A61K  A61P**

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)    **EPO-Internal, WPI Data**

## C. DOCUMENTOS CONSIDERADOS RELEVANTES

| Categoría* | Documentos citados, con indicación, si procede, de las partes relevantes | Relevante para las reivindicaciones N° |
|---|---|---|
| Y | **US 2015/110788 A1 (KIM KYUNG JIN [US] ET AL)** 23 Abril 2015 (2015-04-23) párrafos **[0008], [0018], [0027], [0033], [0040] – [0042], [0054], [0059], [0063], [0064];** reivindicaciones 1,2 ----- | 1-17 |
| Y | **WO 2012/131078 A1 (BOEHRINGER INGELHEIM INT [DE]; GSCHWIND ANDREAS [AT] ET AL.)** 04 Octubre 2012 (2012-10-04) página 5, párrafo 19- página 7, párrafo 28; ejemplos **2,6,8** ----- | 1-17 |
| A | **US 2019/031750 A1 (KOENIG PATRICK [US] ET AL)** 31 Enero 2019 (2019-01-31) párrafos **[0002], [0003], [0033], [0036], [0037], [0041], [0045], [0058], [0183], [0300]** ----- | 1-17 |
| | -/-- | |

| | | |
|---|---|---|
| [X] En la continuación del Recuadro C se relacionan otros documentos | [X] Los documentos de familias de patentes se indican en el Anexo | |

| * | Categorías especiales de documentos citados: | "T" | documento ulterior publicado con posterioridad a la fecha de presentación internacional o de prioridad que no pertenece al estado de la técnica pertinente pero que se cita por permitir la comprensión del principio o teoría que constituye la base de la invención. |
|---|---|---|---|
| "A" | documento que define el estado general de la técnica no considerado como particularmente relevante. | | |
| "E" | solicitud de patente o patente anterior pero publicada en la fecha de presentación internacional o en fecha posterior. | "X" | documento particularmente relevante; la invención reivindicada no puede considerarse nueva o que implique una actividad inventiva por referencia al documento aisladamente considerado. |
| "L" | documento que puede plantear dudas sobre una reivindicación de prioridad o que se cita para determinar la fecha de publicación de otra cita o por una razón especial (como la indicada). | | |
| "O" | documento que se refiere a una divulgación oral, a una utilización, a una exposición o a cualquier otro medio. | "Y" | documento particularmente relevante; la invención reivindicada no puede considerarse que implique una actividad inventiva cuando el documento se asocia a otro u otros documentos de la misma naturaleza, cuya combinación resulta evidente para un experto en la materia. |
| "P" | documento publicado antes de la fecha de presentación internacional pero con posterioridad a la fecha de prioridad reivindicada. | | |
| | | "&" | documento que forma parte de la misma familia de patentes. |

| Fecha en que se ha concluido efectivamente la búsqueda internacional. **11 Enero 2023** | Fecha de expedición del informe de búsqueda internacional **19/01/2023** |
|---|---|
| Nombre y dirección postal de la Administración encargada de la búsqueda internacional    ISA/ **EP** | Funcionario autorizado |
| N° de fax | N° de teléfono |

Formulario PCT/ISA/210 (segunda hoja) (Enero 2015)

## INFORME DE BÚSQUEDA INTERNACIONAL

| | Solicitud internacional N° |
|---|---|
| | **PCT/CU2022/050011** |

| C (continuación). | DOCUMENTOS CONSIDERADOS RELEVANTES | |
|---|---|---|
| Categoría* | Documentos citados, con indicación, si procede, de las partes relevantes | Relevante para las reivindicaciones N° |
| A | US 2009/220523 A1 (SABBADINI ROGER A [US] ET AL) 03 Septiembre 2009 (2009-09-03) párrafos [0029], [0034], [0035], [0074], [0092], [0100] – [0102], [0144] – [0146]<br>----- | 1-17 |

Formulario PCT/ISA/210 (continuación de la segunda hoja) (Enero 2015)

## INFORME DE BÚSQUEDA INTERNACIONAL

| Solicitud internacional N° |
| --- |
| **PCT/CU2022/050011** |

**Recuadro I      Secuencia(s) de nucleótidos y/o de aminoácidos (continuación del punto 1.c de la primera hoja)**

1. En lo que se refiere a las secuencias de nucleótidos y/o de aminoácidos divulgadas en la solicitud internacional y necesarias para la invención reivindicada, la búsqueda se ha llevado a cabo sobre la base de una lista de secuencias:

    a. ☒ que forma parte de la solicitud internacional tal y como se presentó:

        ☐ en formato de archivo de texto según Anexo C/ST.25.

        ☐ en formato papel o en formato de archivo de imagen.

    b. ☐ presentada junto con la solicitud internacional de acuerdo a la Regla 13*ter*.1.a) del PCT exclusivamente a los fines de la búsqueda, en formato de archivo de texto según Anexo C/ST.25.

    c. ☐ Presentada con posterioridad a la fecha de presentación exclusivamente a los fines de la búsqueda internacional:

        ☐ en formato de archivo de texto según Anexo C/ST.25 (Regla 13*ter*.1.a)).

        ☐ en formato papel o en formato de archivo de imagen (Regla 13*ter*.1.b) e Instrucciones Administrativas, sección 713).

2. ☐ Además, en caso de que se haya presentado más de una versión o copia de una lista de secuencias, se ha entregado la declaración requerida de que la información contenida en las copias subsiguientes o adicionales es idéntica a la que formaba parte de la solicitud tal y como se presentó o no va más allá de lo presentado inicialmente

3. Comentarios adicionales:

Formulario PCT/ISA/210 (continuación de la primera hoja(1)) (Enero 2015)

**INFORME DE BÚSQUEDA INTERNACIONAL**

Información relativa a miembros de familias de patentes

| | Solicitud internacional Nº |
|---|---|
| | **PCT/CU2022/050011** |

| | | | | | | | |
|---|---|---|---|---|---|---|---|
| US | 2015110788 | A1 | 23-04-2015 | US | 2015110788 A1 | 23-04-2015 |
| | | | | WO | 2013134414 A1 | 12-09-2013 |
| WO | 2012131078 | A1 | 04-10-2012 | AR | 085984 A1 | 13-11-2013 |
| | | | | AU | 2012237234 A1 | 05-09-2013 |
| | | | | AU | 2017200237 A1 | 02-02-2017 |
| | | | | BR | 112013025304 A2 | 06-06-2017 |
| | | | | CA | 2827817 A1 | 04-10-2012 |
| | | | | CL | 2013002623 A1 | 14-02-2014 |
| | | | | CN | 103562222 A | 05-02-2014 |
| | | | | CN | 105820243 A | 03-08-2016 |
| | | | | CO | 6801639 A2 | 29-11-2013 |
| | | | | CY | 1118339 T1 | 28-06-2017 |
| | | | | DK | 2694546 T3 | 05-12-2016 |
| | | | | EA | 201301108 A1 | 31-03-2014 |
| | | | | EA | 201600338 A1 | 30-09-2016 |
| | | | | EC | SP13013001 A | 31-12-2013 |
| | | | | EP | 2694546 A1 | 12-02-2014 |
| | | | | EP | 3144322 A2 | 22-03-2017 |
| | | | | ES | 2606302 T3 | 23-03-2017 |
| | | | | HK | 1190412 A1 | 04-07-2014 |
| | | | | HK | 1225400 A1 | 08-09-2017 |
| | | | | HR | P20161689 T1 | 24-02-2017 |
| | | | | HU | E030148 T2 | 28-04-2017 |
| | | | | JP | 6023786 B2 | 09-11-2016 |
| | | | | JP | 6297657 B2 | 20-03-2018 |
| | | | | JP | 2014515602 A | 03-07-2014 |
| | | | | JP | 2017023152 A | 02-02-2017 |
| | | | | KR | 20140018317 A | 12-02-2014 |
| | | | | LT | 2694546 T | 10-11-2016 |
| | | | | MA | 34979 B1 | 01-03-2014 |
| | | | | MX | 337543 B | 10-03-2016 |
| | | | | MX | 343440 B | 07-11-2016 |
| | | | | MY | 171007 A | 23-09-2019 |
| | | | | NZ | 614249 A | 29-05-2015 |
| | | | | PE | 20140448 A1 | 13-04-2014 |
| | | | | PL | 2694546 T3 | 31-03-2017 |
| | | | | PT | 2694546 T | 28-12-2016 |
| | | | | SG | 193561 A1 | 29-11-2013 |
| | | | | SG | 10201602373T A | 30-05-2016 |
| | | | | TN | 2013000390 A1 | 20-01-2015 |
| | | | | UA | 114707 C2 | 25-07-2017 |
| | | | | US | 2013078248 A1 | 28-03-2013 |
| | | | | US | 2017247475 A1 | 31-08-2017 |
| | | | | US | 2020010569 A1 | 09-01-2020 |
| | | | | US | 2022017642 A1 | 20-01-2022 |
| | | | | UY | 33998 A | 28-09-2012 |
| | | | | WO | 2012131078 A1 | 04-10-2012 |
| US | 2019031750 | A1 | 31-01-2019 | AU | 2016326666 A1 | 08-02-2018 |
| | | | | BR | 112018005737 A2 | 09-10-2018 |
| | | | | CA | 2992602 A1 | 30-03-2017 |
| | | | | CL | 2018000732 A1 | 22-02-2019 |
| | | | | CL | 2021000519 A1 | 23-07-2021 |
| | | | | CN | 108137681 A | 08-06-2018 |
| | | | | CO | 2018003863 A2 | 10-10-2018 |
| | | | | CR | 20180221 A | 09-07-2018 |
| | | | | EP | 3353203 A2 | 01-08-2018 |

**INFORME DE BÚSQUEDA INTERNACIONAL**

Información relativa a miembros de familias de patentes

Solicitud internacional Nº

**PCT/CU2022/050011**

|  |  |  | HK | 1254198 | A1 | 12-07-2019 |
|---|---|---|---|---|---|---|
|  |  |  | IL | 257565 | A | 30-04-2018 |
|  |  |  | JP | 6959912 | B2 | 05-11-2021 |
|  |  |  | JP | 2018531005 | A | 25-10-2018 |
|  |  |  | JP | 2022017288 | A | 25-01-2022 |
|  |  |  | KR | 20180053315 | A | 21-05-2018 |
|  |  |  | MA | 42924 | A | 01-08-2018 |
|  |  |  | PE | 20181363 | A1 | 27-08-2018 |
|  |  |  | PH | 12018500657 | A1 | 15-10-2018 |
|  |  |  | RU | 2018114723 | A | 23-10-2019 |
|  |  |  | SG | 10201911226Q | A | 30-01-2020 |
|  |  |  | TW | 201718642 | A | 01-06-2017 |
|  |  |  | UA | 125432 | C2 | 09-03-2022 |
|  |  |  | US | 2017096479 | A1 | 06-04-2017 |
|  |  |  | US | 2019031750 | A1 | 31-01-2019 |
|  |  |  | US | 2019100581 | A1 | 04-04-2019 |
|  |  |  | US | 2021115124 | A1 | 22-04-2021 |
|  |  |  | WO | 2017053807 | A2 | 30-03-2017 |
|  |  |  | ZA | 201800770 | B | 26-05-2021 |
| US 2009220523 | A1 | 03-09-2009 | AU | 2006309008 | A1 | 10-05-2007 |
|  |  |  | CA | 2627427 | A1 | 10-05-2007 |
|  |  |  | CN | 101340929 | A | 07-01-2009 |
|  |  |  | DK | 1948234 | T3 | 28-01-2013 |
|  |  |  | EA | 200801197 | A1 | 27-02-2009 |
|  |  |  | EP | 1948234 | A2 | 30-07-2008 |
|  |  |  | ES | 2398919 | T3 | 22-03-2013 |
|  |  |  | HK | 1123000 | A1 | 05-06-2009 |
|  |  |  | JP | 5739089 | B2 | 24-06-2015 |
|  |  |  | JP | 2009513668 | A | 02-04-2009 |
|  |  |  | JP | 2015057382 | A | 26-03-2015 |
|  |  |  | PL | 1948234 | T3 | 28-06-2013 |
|  |  |  | PT | 1948234 | E | 11-02-2013 |
|  |  |  | SI | 1948234 | T1 | 28-02-2013 |
|  |  |  | US | 2006171946 | A1 | 03-08-2006 |
|  |  |  | US | 2007148168 | A1 | 28-06-2007 |
|  |  |  | US | 2009220523 | A1 | 03-09-2009 |
|  |  |  | WO | 2007053447 | A2 | 10-05-2007 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008052489 A, Lamdan, H
- WO 2003035694 A
- WO 2012089176 A

**Non-patent literature cited in the description**

- **FOLKMAN**. *Nat Rev Drug Discov*, 2007, vol. 6, 273-86
- **APTE et al.** *Cell*, 2019, vol. 176, 1248-64
- **JOVČEVSKA ; MUYLDERMANS**. *BioDrugs*, 2020, vol. 34, 11-26
- **MUYLDERMANS**. *FEBS J*, 2021, vol. 288, 2084-102
- **SUN et al.** *Int J Nanomedicine*, 2021, vol. 16, 2337-56
- **SCULLY et al.** *New England Journal of Medicine*, 2019, vol. 380, 335-46
- **MCCAFFERTY et al.** *Nature*, 1990, vol. 348, 552-4
- **KNAPPIK et al.** *Journal of Molecular Biology*, 2000, vol. 296, 57-86
- **WANG et al.** *Molecular Cancer Therapeutics*, 2012, vol. 11, 864-72
- **RONCA et al.** *Expert Opin. Ther. Targets*, 2015, vol. 19, 1-17
- **ZAHRA et al.** *Cancers (Basel)*, 2021, vol. 13, 1422
- **FARAJPOUR et al.** *Journal of Biomolecular Screening*, 2014, vol. 19, 547-55
- **EBRAHIMIZADEH et al.** *Applied Biochemistry and Biotechnology*, 2015, vol. 176, 1985-95
- **KAZEMI-LOMEDASHT et al.** *Molecular Immunology*, 2015, vol. 65, 58-67
- **KAZEMI-LOMEDASHT et al.** *Iranian Journal of Basic Medical Sciences*, 2017, 399-496
- **KAZEMI-LOMEDASHT et al.** *Iranian Journal of Basic Medical Sciences*, 2018, vol. 21, 260-6
- **KOVALCHUK et al.** *Clinical & Experimental Metastasis*, 2020, vol. 37, 637-48
- **SHIBUYA**. *Cell structure and function*, 2001, vol. 26, 25-35
- **SULLIVAN et al.** *PLoS ONE*, 2010, vol. 5, 7-8
- **SELVARAJ et al.** *Cancer Cell*, 2015, vol. 27, 780-96
- **BOURHIS et al.** *Frontiers in Immunology*, 2021, vol. 12, 616837
- **CORTEZ-RETAMOZO et al.** *Int J Cancer*, 2002, vol. 98, 456-62
- **NEZLIN**. *The Immunoglobulins*, 1998, 3-73
- **CONRATH et al.** *Developmental and Comparative Immunology*, 2003, vol. 27, 87-103
- **SAERENS et al.** *Anal Chem*, 2005, vol. 77, 7547-55
- **HAIBE et al.** *Frontiers in Oncology*, 2020, vol. 10, 221
- **CAMPA**. *Curr Drug Targets*, 2020, vol. 21, 1194-200
- **RATH T et al.** *HHS Public Access*, 2016, vol. 35, 235-254
- **HUYVETTER et al.** *Theranostics*, 2014, 4
- **SANCHEZ RAMIREZ et al.** *Journal of immunoassay & immunochemistry*, 2016, vol. 37, 636-58
- **MORERA et al.** *Biotechnology and Applied Biochemistry*, 2006, vol. 44, 45-53
- **LAMDAN et al.** *Journal of Biotechnology*, 2011, vol. 151, 166-74
- **GAVILONDO et al.** *Vaccine*, 2014, vol. 32, 2241-50
- **MARKS et al.** *Journal of Molecular Biology*, 1991, vol. 222, 581-97
- **BEATTY JD ; BEATTY BG ; VLAHOS WG**. *J Immunol Methods.*, 26 June 1987, vol. 100 (1-2), 173-9
- **CONRATH et al.** *Antimicrobial agents and chemotherapy*, 2001, vol. 45, 2807-12
- **VINCKE et al.** *Journal of Biological Chemistry*, 2009, vol. 284, 3273-84
- **KUNKEL**. *Proceedings of the National Academy of Sciences of the United States of America*, 1985, vol. 82, 488-92
- **CABANILLAS-BERNAL et al.** *PLoS One*, 2019, vol. 14, e0213394
- **MARKS et al.** *Journal of molecular biology*, 1991, vol. 222, 581-97
- **SHIBUYA**. *Cell Structure and Function*, 2001, vol. 26, 25-35
- **HMILA et al.** *FASEB Journal*, 2010, vol. 24, 3479-89
- **BEATTY JD ; BEATTY BG ; VLAHOS WG**. *J Immunol Methods*, 26 June 1987, vol. 100 (1-2), 173-9

- **CRAUWELS M** ; **VAN VAERENBERGH N** ; **KULAYA NB et al.** *New Biotechnology*, 2020, vol. 57, 20-28
- **GIANNOS et al.** *Pharmaceutical Research*, 2018, 1-15
- **MUYLDERMANS**. *Annual Review of Biochemistry*, 2013, vol. 82, 17.1-.23
- **MONTESANO, R.** ; **PEPPER, M.S**. Vascular Morphogenesis: In vivo, In vitro, In mente. Birkhauser, 1998, 79-110
- **D. GUIDOLIN**. *Microvascular Research*, 2004, vol. 67, 117-124
- **MORERA et al.** *Exp Eye Res*, 2014, vol. 122, 102-9
- **FRAKER PJ** ; **SPECK JC JR.** *Biochem Biophys Res Comm*, 1978, vol. 80, 849-857
- **BOURHIS et al.** *Frontiers in immunology*, 2021, vol. 12, 616837
- **VORON et al.** *J Exp Med*, 2015, vol. 212, 139-48
- **ASAHARA et al.** *Circulation*, 1995, vol. 92, II365-71
- **IM et al.** *Nature communications*, 2020, vol. 11, 1-14
- **HUGHES et al.** *Molecular Therapy: Methods & Clinical Development*, 2019, vol. 13, 86-98
- **AURNHAMMER et al.** *Hum Gene Ther Methods*, 2012, vol. 23, 18-28